# EUROPEAN PATENT SPECIFICATION

(11) **EP 4 608 400 B1**
(45) Date of publication and mention of the grant of the patent: **19.08.2026**
(21) Application number: 23798734.2
(22) Date of filing: 27.10.2023
(51) Int. Cl.: A61K 31/46, A61K 45/06, A61P 25/02, A61P 25/04, A61P 25/06

(54) **COMPOUND FOR TREATMENT OF PAIN**
VERBINDUNG ZUR SCHMERZBEHANDLUNG
COMPOSÉ POUR LE TRAITEMENT DE LA DOULEUR

(30) Priority: 28.10.2022 EP 22204453
(43) Date of publication of application: 03.09.2025
(73) Proprietor: Initiator Pharma A/S, 2200 Copenhagen N (DK)
(72) Inventor: SIMONSEN, Ulf, 2200 Copenhagen N (DK); THOMSEN, Mikael, 2200 Copenhagen N (DK)
(74) Representative: Holm, Peter Joakim
(86) International application number: PCT/EP2023/080077
(87) International publication number: WO 2024/089247

(56) References cited:
- WO-A1-2011/092061
- CAVALLI EUGENIO ET AL: "The neuropathic pain: An overview of the current treatment and future therapeutic approaches", INTERNATIONAL JOURNAL OF IMMUNOPATHOLOGY AND PHARMACOLOGY VOLUME, 22 March 2019 (2019-03-22), pages 1 - 10, XP093035244, Retrieved from the Internet <URL:https://www.ncbi.nlm.nih.gov/pmc/articles/PMC6431761/pdf/10.1177_2058738419838383.pdf> [retrieved on 20230328], DOI: 10.1177/2058738419838383

## Description

### Technical field

The present disclosure relates to a compound of formula (I) as described herein for use in the treatment, prevention, or alleviation of pain in a subject, wherein the compound is administered in an amount of 0.5 mg to 10 mg per individual dose.

### Background

Neuropathic pain occurs after a lesion or injury to the somatosensory system and significantly impacts the quality of life. Examples include trigeminal neuralgia, painful polyneuropathy, postherpetic neuralgia, and central poststroke pain. Evoked pain may spread to neighboring areas and involve peripheral and central sensitization. The prevalence of neuropathic pain is 6.9 to 10%, often affecting people with diabetes. Currently, 200 million people worldwide suffer from neuropathic pain, also known as diabetic polyneuropathy.

Anticonvulsants, e.g., carbamazepine, oxcarbazepine, and topiramate with an effect on sodium channels, are generally not recommended. The same applies to other anticonvulsants, e.g., lamotrigine, lacosamide, phenytoin, and levetiracetam. Tri-and tetracyclic antidepressants are recommended as a first-line treatment for neuropathic pain. Still, risk-benefit assessment must consider the side effects, drug interactions, and cardiac toxicity of these drugs.

The selective serotonin- and noradrenaline-reuptake inhibitor duloxetine is recommended as a first-choice treatment. It has shown a comparable effect to amitriptyline and pregabalin in patients with diabetic neuropathy. Serious adverse effects are rare. However, side effects may occur, including nausea, fatigue, dizziness, increased sweating, dry mouth, constipation, reduced appetite, insomnia, diarrhoea, disturbed consciousness and trembling, and an increase in intraocular pressure and high blood pressure (BP). Moreover, duloxetine is converted by cytochrome P450 (CYP) 1A2 and interacts with ciprofloxazin and metoprolol; it cannot be combined with serotonergic drugs.

Local treatments, including lidocaine, capsaicin, and botulinum toxin, are considered for the treatment of neuropathic pain. However, inadequate response to drug treatments constitutes a substantial unmet need in patients with neuropathic pain, and currently, less than one-third of painful diabetic neuropathy patients derive sufficient pain relief with existing pharmacotherapies.

Inadequate response to drug treatments constitutes a substantial unmet need in patients with neuropathic pain. Therefore, there is a marked unmet need for new treatment modalities for neuropathic pain.

WO 2011/092061 A1 discloses 8-aza-bicyclo[3.2.1]oct-3-yloxy)-chromen-2-one derivatives useful as monoamine neurotransmitter reuptake inhibitors.

### Summary

As outlined above, a compound able to treat neuropathic pain is highly desired. The present disclosure provides for a compound useful in the treatment of pain, such as neuropathic pain.

Thus, in one main aspect, the present disclosure provides a compound of formula (I), 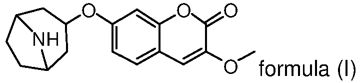 or a pharmaceutically acceptable salt thereof, for use in the treatment, prevention or alleviation of pain in a subject, wherein the compound is administered in an amount of 0.5 mg to 10 mg per individual dose.

The inventors have surprisingly shown that the use of a compound of formula I as described herein has antinociceptive effects in different models of pain, including neuropathic pain. The inventors have surprisingly found that the use of the compound has anti-nociceptive effects in different models of neuropathic injury in mammals, including humans, and that the compound for use as described herein effectively treats signs of spontaneous, ongoing pain after neuropathic injury.

In humans, the inventors have surprisingly shown that the use of the compound of formula I improves the rating of different parameters such as subjective rating of pain, allodynia and hyperalgesia after neuropathic injury provoked by intradermal injection of capsaicin.

The inventors have surprisingly demonstrated in studies in healthy volunteers that doses of the compound of 10 mg or below are linked to a lower incidence of treatment-emergent adverse effects (TEAEs) compared to higher doses.

These findings demonstrate completely a new way to treat neuropathic pain using the compound of the present disclosure, with dosage regimes that are effective but have lower risk of adverse events, potentially leading to significantly improved quality-of-life for patients suffering from neuropathic pain in different forms.

The invention is defined by the claims. Any subject matter falling outside the scope of the claims is provided for information purposes only.

The references to methods of treatment in this description are to be interpreted as references to the compounds, pharmaceutical compositions and medicaments of the present invention for use in a method for the treatment of the human (or animal) body by therapy.

### Description of Drawings

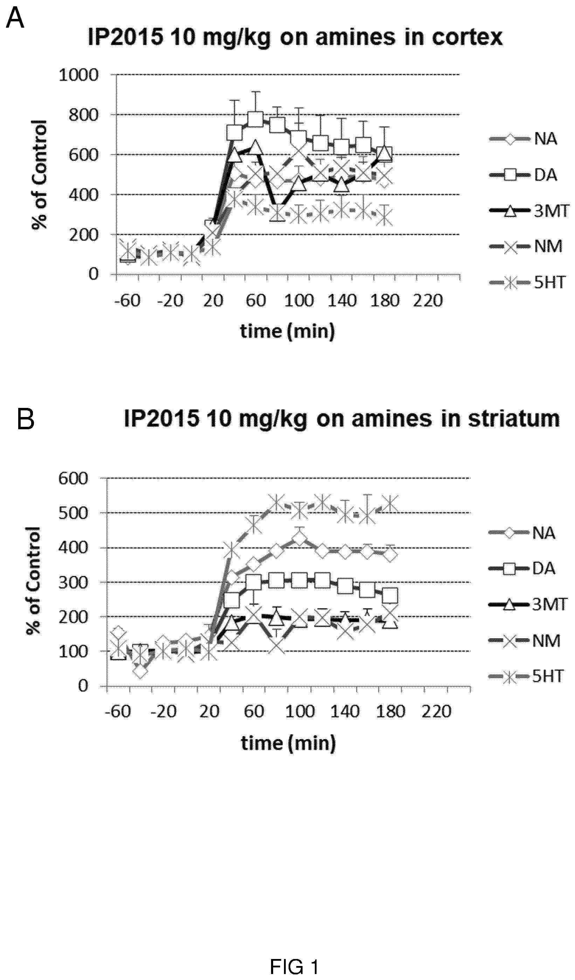

Figure 1. Microdialysis studies in (A) cortex and (B) striatum performed after injection of IP2015 10mg/kg in mice. Dopamine (DA) elevation is most pronounced in cortex, while there is only some elevation of DA in striatum. NA=noradrenaline NM = NE-metabolite (normetanephrine); 3MT = DA-metabolite (3-methoxytyramine ;5-HT,5-hydroxytryptami ne. The numbers (n) of animals were 1-2. Please note different scale of y-axis in (A) vs. (B).

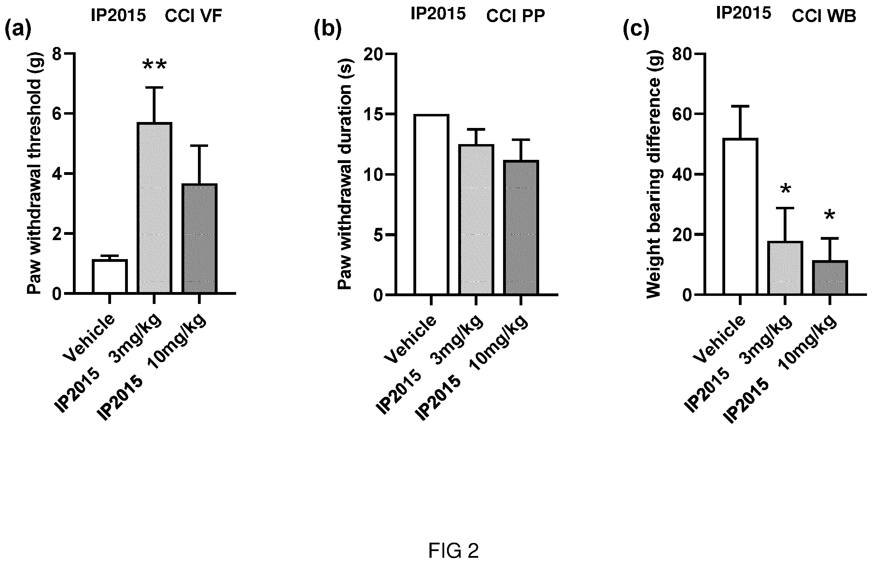

Figure 2. IP2015 diminishes nociceptive behaviours in CCI rats. Adult male rats were injected with either vehicle or IP2015 (3, 10 mg/kg, s.c.) 60 min prior to assessing hindpaw nociceptive behaviours. (a) hindpaw withdrawal threshold (g) to low threshold von Frey stimulation; pre- vs post-CCI baseline = 17.3 vs 1.4 g (b) hindpaw withdrawal duration (s) to high threshold pin prick stimulation; pre- vs post-CCI baseline = 0 vs 14.9 s (c) weight bearing difference (g) as a surrogate index of spontaneous, ongoing pain; - 9.6 vs 42.1 g. All groups n=8 rats. Data are presented as mean ± S.E.M. (a) F(2,21)=5.302 P=0.0137, (b) F(2,21)=2.588, P=0.0989, (c) F(2,21)=5.118, P=0.0155; *P<0.05, **P<0.01 vs corresponding Vehicle; One Way ANOVA followed by Dunnett's test.

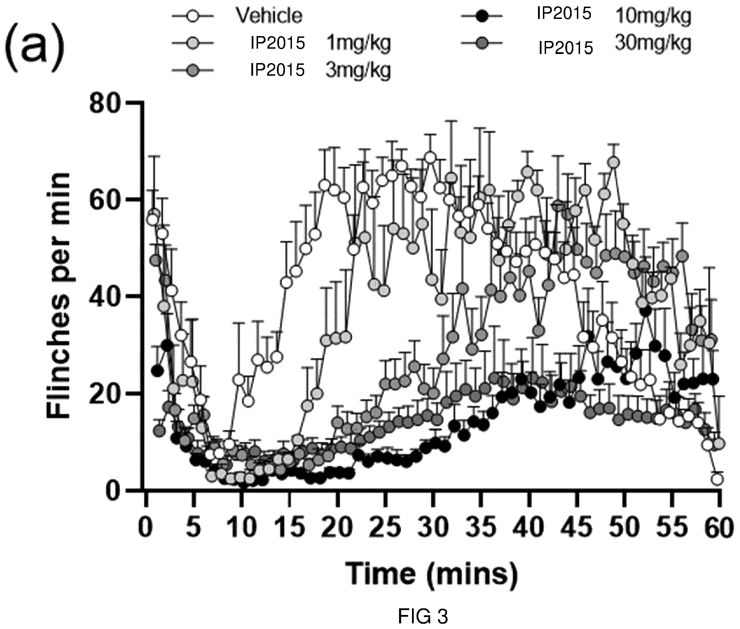

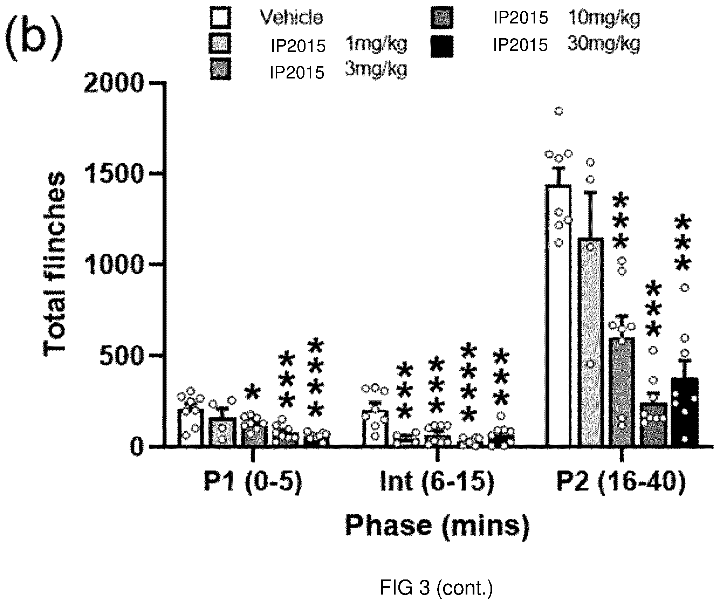

Figure 3. IP2015 diminishes nociceptive behaviours in the formalin test. Adult male rats were injected with either vehicle or IP2015 (1, 3, 10, 30 mg/kg, s.c.) 60 min prior to injection of 5% formalin into the dorsal surface of the hindpaw. (a) time course of flinching behaviour after formalin injection (b) total flinches are shown for first phase (P1=0-5 min), interphase (Int=6-15 min), second phase (P2=16-40 min) of the test after administration of formalin. All groups n=8 rats, except IP2015 1 mg/kg n=4 rats. Data are presented as mean ± S.E.M. P1 F(4,31)=7.944, P=0.0022; Int F(4,31)=10.23, P<0.0001; P2 F(4,31)=23.07; P<0.0001 *P<0.05, ***P<0.001, ****P<0.0001 vs corresponding Vehicle; One Way ANOVA followed by Dunnett's test.

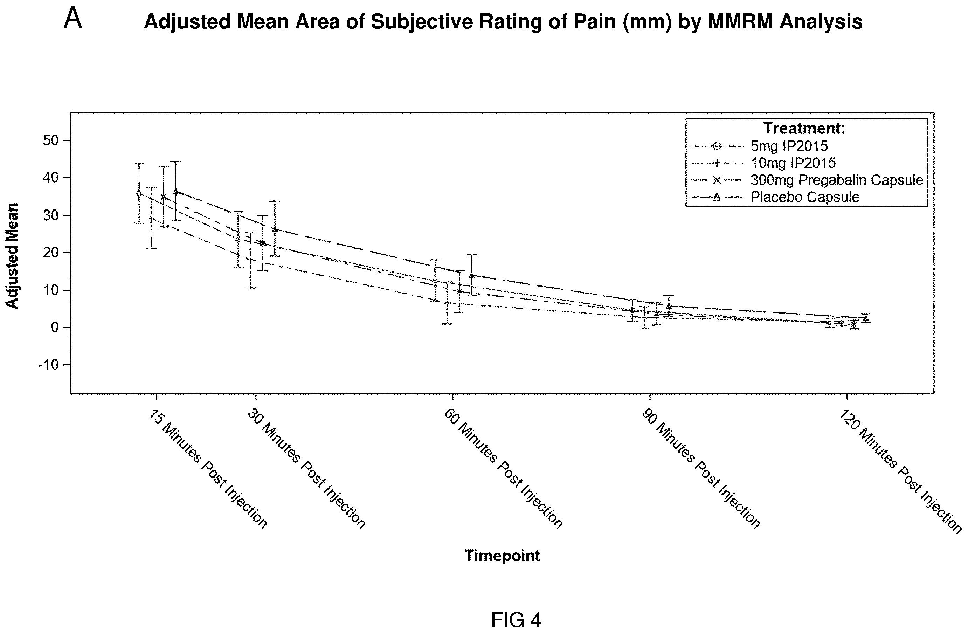

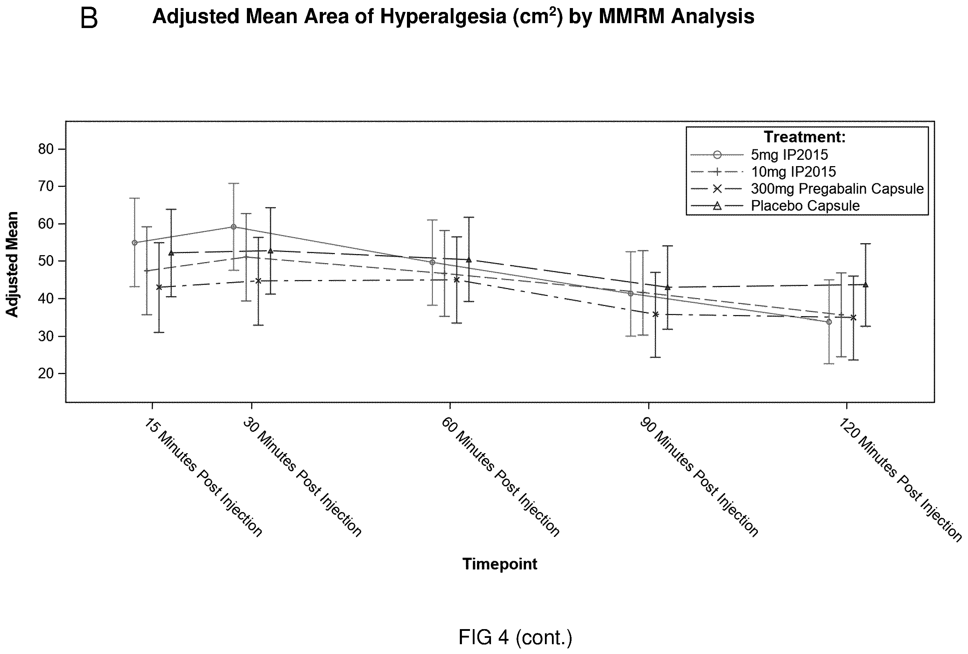

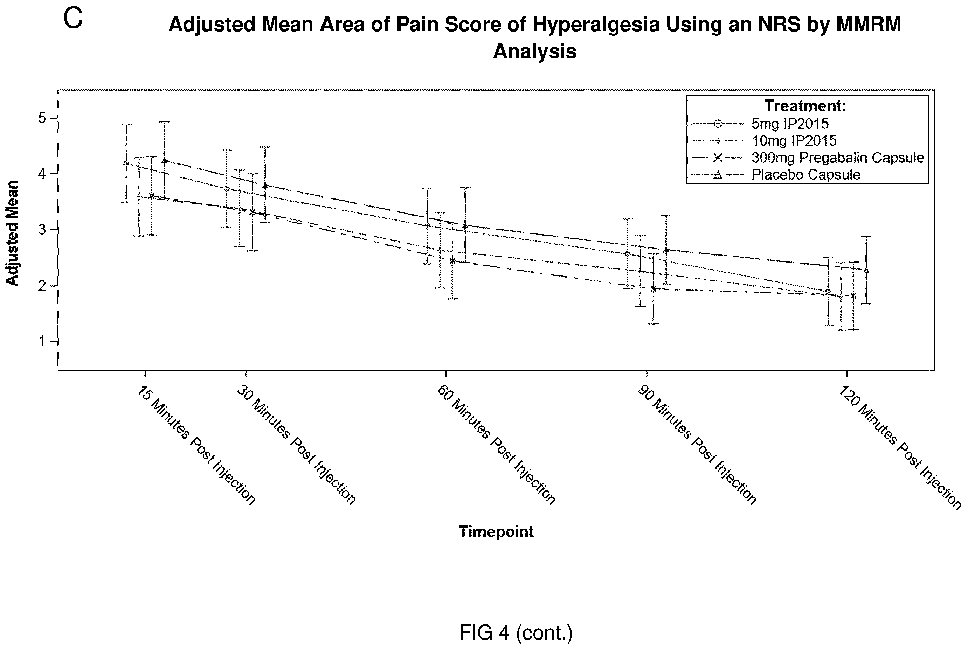

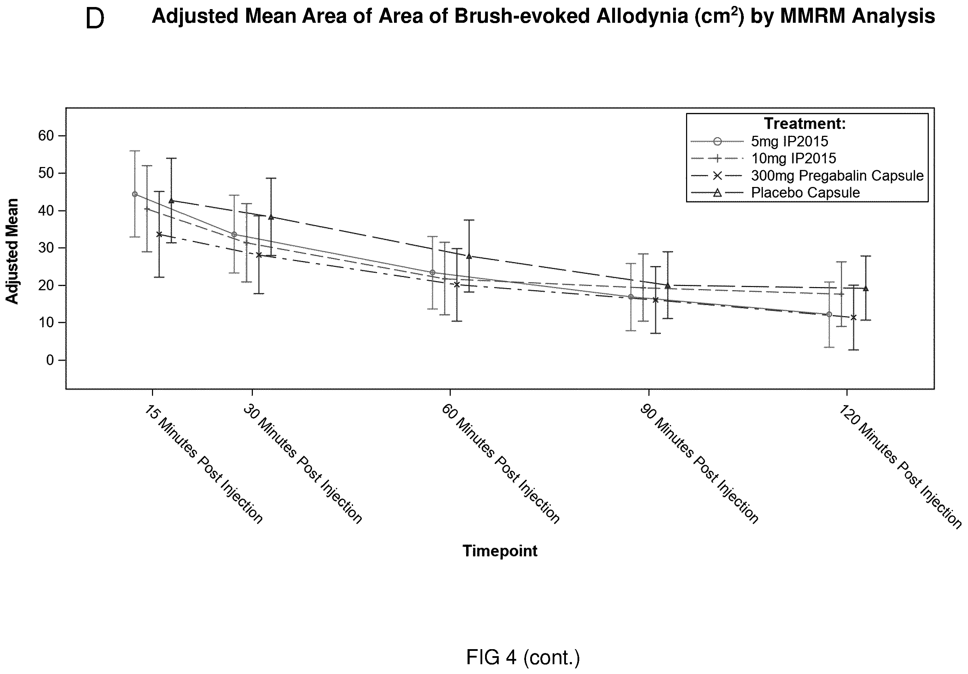

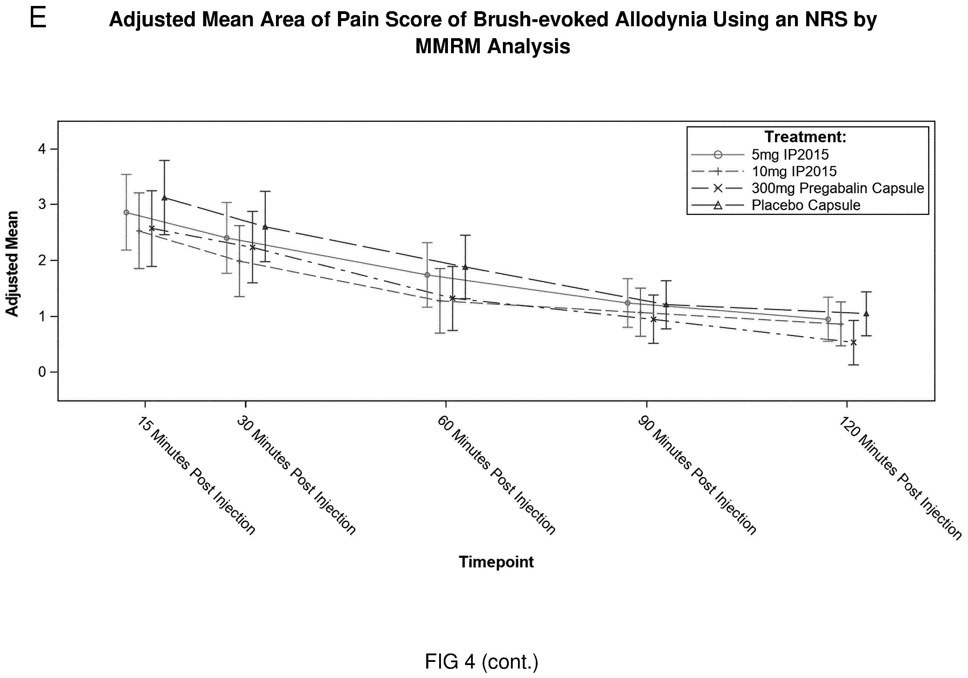

Figure 4. (A) Adjusted Mean Area of Subjective Rating of Pain (mm) by MMRM Analysis (B) Adjusted mean area of hyperalgesia (cm²) by MMRM analysis; (C) Adjusted Mean Area of Pain Score of Hyperalgesia Using an NRS by MMRM Analysis; (D) Adjusted Mean Area of Area of Brush-evoked Allodynia (cm²) by MMRM Analysis; (E) Adjusted Mean Area of Pain Score of Brush-evoked Allodynia Using an NRS by MMRM Analysis. Abbreviations: MMRM - mixed model repeated measures. Error bars represent the 95% confidence interval of adjusted mean from MMRM model

### Definitions

"Pharmaceutically acceptable" means that which is useful in preparing a pharmaceutical composition that is generally safe, non-toxic, and neither biologically nor otherwise undesirable and includes that which is acceptable for veterinary as well as human pharmaceutical use.

The term "pharmaceutically acceptable salt" of a compound refers to a salt that is pharmaceutically acceptable, as defined herein, and preferably possesses the desired pharmacological activity of the parent compound. Pharmaceutically acceptable salts include acid addition salts formed with inorganic acids, e.g. hydrochloric acid, hydrobromic acid, sulfuric acid, nitric acid, phosphoric acid; or formed with organic acids, e.g. acetic acid, benzenesulfonic acid, benzoic acid, camphorsulfonic acid, citric acid, ethanesulfonic acid, fumaric acid, glucoheptonic acid, gluconic acid, glutamic acid, glycolic acid, hydroxynaphtoic acid, 2-hydroxyethanesulfonic acid, lactic acid, maleic acid, malic acid, malonic acid, mandelic acid, methanesulfonic acid, muconic acid, 2-naphthalenesulfonic acid, propionic acid, salicylic acid, succinic acid, tartaric acid, p-toluenesulfonic acid, trimethylacetic acid; or salts formed when an acidic proton present in the parent compound either is replaced by a metal ion, e.g., an alkali metal ion, an alkaline earth ion, or an aluminium ion; or coordinates with an organic or inorganic base. Acceptable organic bases include e.g. diethanolamine, ethanolamine, N-methylglucamine, triethanolamine, morpholine, and tromethamine. Acceptable inorganic bases include e.g. ammonia, aluminum hydroxide, calcium hydroxide, potassium hydroxide, sodium carbonate and sodium hydroxide.

As used herein the terms "treatment" or" treating" is an approach for obtaining beneficial or desired results including clinical results. Beneficial or desired clinical results can include, but are not limited to, alleviation or amelioration of one or more symptoms or conditions, diminishment of extent of disease or disorder, stabilized (i.e., not worsening) state of disease or disorder, prevention of the disease or disorder, delay or slowing of disease or disorder progression, amelioration or palliation of the disease state, and remission (whether partial or total) whether detectable or undetectable.

Whenever a chiral carbon is present in a chemical structure, it is intended that all stereoisomers associated with that chiral carbon are encompassed by the structure, unless otherwise specified. Using the Cahn-Ingold-Prelog RS notational system, any asymmetric carbon atom may be present in the (R)- or (S)-configuration, and the compound may be present as a mixture of its stereoisomers, e.g. a racemic mixture, or one stereoisomer only.

The compound for use according to the present disclosure may exist in a tautomeric form. Any such tautomer is considered to be within the scope of the compound for use according to the present disclosure.

Also, in the compound of formula I as defined herein, any hydrogen atom may be replaced by a deuterium (²H), and any such deuterated compound of formula I, comprising one or more deuterium atoms in place of the corresponding number of hydrogen atoms, is considered to be within the scope of the compound for use according to the present disclosure.

It is known in the art that prodrugs can be produced. The person skilled in the art will know which types of molecular moieties can be introduced on a drug to produce a prodrug. It is considered that prodrugs relating to the compound of formula I are within the scope of the compound for use according to the present disclosure.

As used herein, "neuropathic pain" includes reference to a neuropathic component of nociceptive pain.

As used herein, "nociceptive" means relating to the perception or sensation of pain.

By "IP2015" or "compound I" is meant the compound of formula I. The compound of formula I is also known as pudafensine. The compound of formula I is:

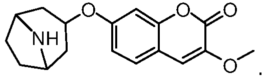

As used herein, "formulation" is the result of combining different substances, including the active ingredient, to produce a final product.

The term "about" as used herein to refer to an amount or percentage is to be interpreted as a variation of ± 10% with respect to the value of the amount or percentage it refers to, such as ± 5%.

### Detailed description

### Compounds for use

One embodiment of the present disclosure provides for a compound of formula (1), 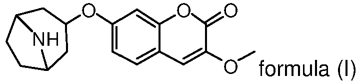 or a pharmaceutically acceptable salt thereof for use in the treatment, prevention or alleviation of pain in a subject, wherein the compound is administered in an amount of 0.5 mg to 10 mg per individual dose. The compound of the present disclosure is a monoamine reuptake inhibitor. Compounds may be tested for their ability to inhibit reuptake of the monoamines dopamine, noradrenaline and serotonin in synaptosomes e.g. such as described in WO 97/30997 or WO 97/16451. The compound of formula (I) hydrochloride has been reported with the IC₅₀ values: 0.0029 µM (serotonin) 0.07 µM (dopamine) 0.0038 µM (noradrenaline) (US 9,133,184 B1).

In one embodiment, the compound of formula (I) has the structure of formula (la); 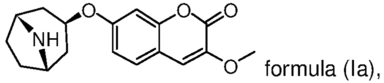 or a pharmaceutically acceptable salt thereof.

In one embodiment, the compound is 7-[(8-azabicyclo[3.2.1]octan-3-yl)oxy]-3-methoxy-chromen-2-one, or a pharmaceutically acceptable salt thereof. **In** one embodiment, the compound is exo-7-[(8-azabicyclo[3.2.1]octan-3-yl)oxy]-3-methoxy-chromen-2-one, or a pharmaceutically acceptable salt thereof. In one embodiment, the name "IP2015" means the compound of formula I. In one embodiment, the name "IP2015" means the compound of formula la. In a specific embodiment of the present disclosure "IP2015" means the hydrochloride of the compound of formula I.

In one embodiment, the pharmaceutically acceptable salt is of an organic or an inorganic counterion. In one embodiment, the pharmaceutically acceptable salt is selected from the list consisting of the hydrochloride, the hydrobromide, the nitrate, the perchlorate, the phosphate, the sulphate, the formate, the acetate, the aconate, the ascorbate, the benzenesulphonate, the benzoate, the cinnamate, the citrate, the embonate, the enantate, the fumarate, the glutamate, the glycolate, the lactate, the maleate, the malonate, the mandelate, the methanesulphonate, the naphthalene-2-sulphonate, the phthalate, the salicylate, the sorbate, the stearate, the succinate, the tartrate, the toluene-p-sulphonate. Other pharmaceutically acceptable salts are known to those of skill in the art. Such salts may be formed by procedures well known and described in the art.

In one embodiment of the disclosure, the compound is exo-7-[(8-azabicyclo[3.2.1]octan-3-yl)oxy]-3-methoxy-chromen-2-one hydrochloride.

The compound of the present disclosure is a monoamine reuptake inhibitor. Compounds may be tested for their ability to inhibit reuptake of the monoamines dopamine, noradrenaline and serotonin in synaptosomes e.g. such as described in WO 97/30997 or WO 97/16451. The compound of formula (I) hydrochloride has been reported with the IC₅₀ values: 0.0029 µM (serotonin) 0.07 µM (dopamine) 0.0038 µM (noradrenaline) (US 9,133,184 B1).

One embodiment of the disclosure provides for a compound of formula I, 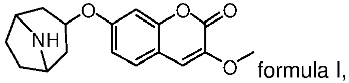 or a pharmaceutically acceptable salt thereof, for use in the treatment, prevention or alleviation of pain in a subject, wherein the compound is administered in an amount of 0.5 mg to 10 mg per individual dose. The examples disclosed herein shows that the compound of formula I is useful for the treatment of pain, such as nociceptive pain and/or neuropathic pain.

In one embodiment of the present disclosure the compound of formula I, 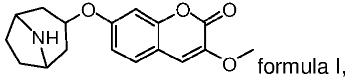 or a pharmaceutically acceptable salt thereof, for use as described herein, is administered in a dose from about 0.001 mg/kg to about 1 mg/kg.

In one embodiment, the compound of formula I is administered in an amount per individual dose from about 0.001 mg/kg to about 1 mg/kg, such as 0.005 mg/kg, such as 0.01 mg/kg, such as 0.015 mg/kg, such as 0.02 mg/kg, such as 0.025 mg/kg, such as 0.03 mg/kg, such as 0.035 mg/kg, such as 0.04 mg/kg, such as 0.045 mg/kg, such as 0.05 mg/kg, such as 0.055 mg/kg, such as 0.06 mg/kg, such as 0.065 mg/kg, such as 0.07 mg/kg, such as 0.075 mg/kg, such as 0.08 mg/kg, such as 0.085 mg/kg, such as 0.09 mg/kg, such as 0.095 mg/kg, such as 0.1mg/kg, such as 0.15 mg/kg, such as 0.2 mg/kg, such as 0.25 mg/kg, such as 0.3 mg/kg, such as 0.35 mg/kg, such as 0.4 mg/kg, such as 0.45 mg/kg, such as 0.5 mg/kg, such as 0.55 mg/kg, such as 0.6 mg/kg, such as 0.65 mg/kg, such as 0.7 mg/kg, such as 0.75 mg/kg, such as 0.8 mg/kg, such as 0.85 mg/kg, such as 0.9 mg/kg, such as 0.95 mg/kg, such as 1 mg/kg per individual dose.

In one embodiment, the compound is administered orally in an amount per individual dose from about 0.01 mg/kg to about 1 mg/kg, such as 0.01 mg/kg, such as 0.05 mg/kg, such as 0.1 mg/kg, such as 0.15 mg/kg, such as 0.2 mg/kg, such as 0.25 mg/kg, such as 0.3 mg/kg, such as 0.35 mg/kg, such as 0.4 mg/kg, such as 0.45 mg/kg, such as 0.5 mg/kg, such as 0.55 mg/kg, such as 0.6 mg/kg, such as 0.65 mg/kg, such as 0.7 mg/kg, such as 0.75 mg/kg, such as 0.8 mg/kg, such as 0.85 mg/kg, such as 0.9 mg/kg, such as 0.95 mg/kg, such as 1 mg/kg per individual dose.

In one embodiment, the compound is administered intravenously in an amount per individual dose from about 0.01 to about 1 mg/kg, such as 0.001 mg/kg, such as 0.005 mg/kg, such as 0.01 mg/kg, such as 0.015 mg/kg, such as 0.02 mg/kg, such as 0.025 mg/kg, such as 0.03 mg/kg, such as 0.035 mg/kg, such as 0.04 mg/kg, such as 0.045 mg/kg, such as 0.05 mg/kg, such as 0.055 mg/kg, such as 0.06 mg/kg, such as 0.065 mg/kg, such as 0.07 mg/kg, such as 0.075 mg/kg, such as 0.08 mg/kg, such as 0.085 mg/kg, such as 0.09 mg/kg, such as 0.095 mg/kg, such as 0.1 mg/kg per individual dose.

In one embodiment, the compound is administered in an amount of 1 mg, 2 mg, 3 mg, 4 mg, 5 mg, 6 mg, 7 mg, 8 mg, 9 mg, or 10 mg per individual dose.

The inventors have surprinsgly shown in healthy volunteers that single doses of 0.01 to 10 mg of the compound of formula I are associated with a lower incidence of treatment-emergent adverse effects compared to single doses of 16.2 mg. Thus, in one embodiment, the compound is administered in an amount from 0.5 to 10 mg per individual dose, such as from 1 to 10 mg per individual dose, such as 1, 2, 3, 4, 5, 6, 7, 8, 9, or 10 mg per individual dose. In one embodiment, the compound is administered in an amount from 5 to 10 mg per individual dose. These dosage regimes are advantageous as they are associated with a lower incidence of treatment-emergent adverse events.

In one embodiment, the compound is administered more than once a day, such as 2 times a day, such as 3 times a day, such as 4 times a day, such as 5, 6, 7 or 8 times a day.

In one embodiment, the compound is administered once daily. In one embodiment, the compound is administered orally.

In one embodiment, the total daily dose of the compound of formula I is from about 0.5 mg to 100 mg, such as 0.6mg, 1mg, 2mg, 3mg, 4mg, 5mg, 6mg, 7mg, 8mg, 9mg, 10mg, 11mg, 12mg, 13mg, 14mg, 15mg, 16mg, 17mg, 18mg, 19mg, 20mg, 21mg, 22mg, 23mg, 24mg, 25mg, 26mg, 27mg, 28mg, 29mg, 30mg, 31mg, 32mg, 33mg, 34mg, 35mg, 36mg, 37mg, 38mg, 39mg, 40mg, 41mg, 42mg, 43mg, 44mg, 45mg, 46mg, 47mg, 48mg, 49mg, 50mg, 51mg, 52mg, 53mg, 54mg, 55mg, 56mg, 57mg, 58mg, 59mg, 60mg, 61mg, 62mg, 63mg, 64mg, 65mg, 66mg, 67mg, 68mg, 69mg, 70mg, 71mg, 72mg, 73mg, 74mg, 75mg, 76mg, 77mg, 78mg, 79mg, 80mg, 81mg, 82mg, 83mg, 84mg, 85mg, 86mg, 87mg, 88mg, 89mg, 90mg, 91mg, 92mg, 93mg, 94mg, 95mg, 96mg, 97mg, 98mg, or 99 mg in total daily dose.

In one embodiment, the compound is administered during at least a day. In one embodiment, the compound is administered during at least 3 days. In one embodiment, the compound is administered during at least 5 days. In one embodiment, the compound is administered at least a week. In one embodiment, the compound is administered at least two weeks. In one embodiment, the compound is administered at least three weeks. In one embodiment, the compound is administered for at least a month. In one embodiment, the compound is administered for at least 3 months.

One embodiment of the disclosure provides for a compound of formula I, 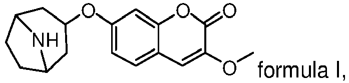 or a pharmaceutically acceptable salt thereof, for use in the treatment, prevention or alleviation of pain in a subject, for example the treatment, prevention or alleviation acute pain, chronic pain, mild pain, moderate or severe pain, postoperative pain, neuropathic pain, central neuropathic pain, pain related to diabetic neuropathy, to postherpetic neuralgia, to peripheral nerve injury, to phantom limb pain, to restless leg syndrome, to neurogenic inflammation, to fibromyalgia, to chronic regional pain syndrome, somatic pain, visceral pain or cutaneous pain, pain caused by inflammation or by infection, pain related to arthritis, osteoarthritis, rheumatoid arthritis, neuronal hyperexcitability disorders, peripheral nerve hyperexcitability, back pain, cancer pain, dental pain, irritable bowel pain, irritable bowel syndrome, post-operative pain, post-mastectomy pain syndrome (PMPS), post-stroke pain, drug-induced neuropathy, complex regional pain syndrome (CRPS), sympathetically maintained pain (SMP), trigeminal neuralgia, myofacial pain, chronic headache, migraine, migraine-related disorders or tension-type headache; wherein the compound is administered in a dose from 0.5 to 10 mg per individual dose.

"Pain" is an unpleasant sensory and emotional experience associated with, or resembling that associated with actual or potential tissue damage. "Chronic pain" is pain that persists or recurs for longer than 3 months. "Acute pain" refers to pain that persists or recurs for a duration of less than 3 months. "Primary pain" is used to describe pain that it is not caused by a different medical condition. "Secondary pain" is used to describe pain that is a consequence of another condition.

In one embodiment, the pain is chronic pain. In one embodiment, the pain is acute pain. In one embodiment, the pain is primary pain. In one embodiment, the pain is secondary pain.

In one embodiment, the pain is selected from the group consisting of:
(i) chronic or acute primary pain,
(ii) chronic or acute cancer related pain,
(iii) chronic or acute postsurgical pain,
(iv) chronic or acute post-traumatic pain,
(v) chronic or acute secondary musculoskeletal pain,
(vi) chronic or acute secondary visceral pain,
(vii) chronic or acute neuropathic pain, and
(viii) chronic or acute secondary headache or orofacial pain.

In one embodiment, the pain is chronic or acute primary pain. In one embodiment, the chronic or acute primary pain is chronic or acute widespread pain, fibromyalgia syndrome, chronic or acute primary musculoskeletal pain, chronic or acute primary headache or orofacial pain, such as migraine, burning mouth syndrome, tension-type headache, cluster headache or hemicranias continua; complex regional pain syndrome (CRPS) or painful bruising syndrome.

In one embodiment, the pain is chronic or acute cancer-related pain. In one embodiment, the chronic or acute cancer-related pain is related to visceral cancer pain, bone cancer pain or neuropathic cancer pain. In one embodiment, the chronic or acute cancer-related pain is post cancer medicine pain, such as painful chemotherapy-induced polyneuropathy, such as post radiotherapy pain, such as painful radiation-induced neuropathy.

In one embodiment, the pain is chronic or acute postsurgical pain. In one embodiment, the chronic or acute postsurgical pain is postsurgical pain after spinal surgery, after herniotomy, after hysterectomy, after amputation, after thoracotomy, after breast surgery or after arthroplasty.

In one embodiment, the pain is chronic or acute post-traumatic pain. In one embodiment, the chronic or acute post-traumatic pain is pain after burns injury, pain associated to whiplash injury, pain after musculoskeletal injury.

In one embodiment, the pain is chronic or acute secondary musculoskeletal pain. In one embodiment, the chronic or acute secondary musculoskeletal pain is pain form persistent inflammation, such as inflammation due to infection, inflammation due to crystal deposition or inflammation due to autoimmune and auto-inflammatory disorders; pain associated with structural changes, such as pain associated to osteoarthritis or associated with spondylosis; pain due to disease of the nervous system such as pain associated to Parkinson's disease, such as pain associated to multiple sclerosis or pain associated to peripheral neurologic disease.

In one embodiment, the pain is chronic or acute secondary visceral pain. In one embodiment, the chronic or acute secondary visceral pain is pain from mechanical factors, vascular mechanism or persistent inflammation, such as mechanical factor, vascular mechanism or persistent inflammation in the head, neck, thoracic, abdominal or pelvic regions.

In one embodiment, the pain is chronic or acute neuropathic pain. In one embodiment, the chronic or acute neuropathic pain is central neuropathic pain, such as central neuropathic pain associated with spinal cord injury, brain injury, post stroke pain or associated with multiple sclerosis; peripheral neuropathic pain, peripheral neuropathic pain after nerve injury, painful polyneuropathy or painful radiculopathy; or neuropathic orofacial pain.

In one embodiment, the pain is peripheral neuropathic pain.

In one embodiment, the pain is chronic or acute secondary headache or orofacial pain. In one embodiment, the chronic or acute secondary headache or orofacial pain is chronic dental pain, chronic neuropathic orofacial pain, headache or orofacial pain associated with temporomandibular disorders, associated with disorders in homeostasis or their nonpharmacological treatment, associated with cranial or cervical vascular disorder, associated with non-vascular intracranial disorder, associated with a substance or its withdrawal, or associated with traumatic injury to the head.

In one embodiment, the pain is neuropathic pain. "Neuropathic pain" is caused by a lesion or disease of the somatosensory nervous system. It can be described as electric, burning, or shock like. The pain may occur spontaneously, without provocation, or be provoked by noxious or nonnoxious stimuli. The pain may be constant or intermittent, and may be described as searing, burning, or icy cold. Neuropathic pain can originate from several different sources.

In one embodiment, the pain is chronic neuropathic pain. In one embodiment, the pain is acute neuropathic pain.

Neuropathic pain can originate from several different sources. In one embodiment, the neuropathic pain is due to alcoholism, diabetes, multiple sclerosis, multiple myeloma, stroke, cancer, cytomegalovirus, trigeminal neuralgia, spinal cord injury, or amputation. In one embodiment, the neuropathic pain is due to chronic or progressive nerve disease.

In one embodiment, the neuropathic pain is due to infection. In one embodiment, the neuropathic pain is due to injury. In one embodiment, the neuropathic pain is ongoing pain after neuropathic injury. In one embodiment, the neuropathic pain is due to a side effect of a medication. In one embodiment, the neuropathic pain is due to a medical procedure, such as surgery.

In one embodiment, the neuropathic pain is idiopathic. In one embodiment, the neuropathic pain is a neuropathic component of nociceptive pain.

In one embodiment, the neuropathic pain is due to diabetic neuropathy. In one embodiment, the diabetes is chronic diabetes.

In one embodiment, the neuropathic pain is neuropathic cancer pain.

In one embodiment, the neuropathic pain is due to treatment-emergent neuropathy. In one embodiment, the treatment-emergent neuropathy is due to surgery.

In one embodiment, the pain is orofacial pain.

In one embodiment, the pain is trigeminal neuralgia. "Trigeminal neuralgia" is a manifestation of orofacial neuropathic pain restricted to one or more divisions of the trigeminal nerve. The pain is recurrent, abrupt in onset and termination, triggered by innocuous stimuli and typically compared to an electric shock or described as shooting or stabbing. Some patients experience continuous pain between these painful paroxysms.

In one embodiment, the pain is classical trigeminal neuralgia. In one embodiment, the pain is secondary trigeminal neuralgia, such as attributed to multiple sclerosis, such as attributed to a space-occupying lesion. In one embodiment, the trigeminal neuralgia is idiopathic trigeminal neuralgia.

In one embodiment, the pain is post-herpetic neuralgia. In one embodiment, the pain is pain in Parkinson's disease.

In one embodiment, the pain is allodynia. "Allodynia" refers to pain resulting from stimulus which would not normally provoke pain, such as a light touch of the skin.

In one embodiment, the pain is hyperalgesia. "Hyperalgesia" refers to excessive sensitivity to painful stimuli.

In one embodiment, the pain is sexual pain disorder. Sexual pain disorders refer to marked and persistent or recurrent difficulties related to the experience of pain during sexual activity in adults, which are not entirely attributable to an underlying medical condition, insufficient lubrication in women, age-related changes, or changes associated with menopause in women and are associated with clinically significant distress.

In one embodiment, the sexual pain disorder is sexual pain-penetration disorder. Sexual pain-penetration disorder is characterised by at least one of the following: 1) marked and persistent or recurrent difficulties with penetration, including due to involuntary tightening or tautness of the pelvic floor muscles during attempted penetration; 2) marked and persistent or recurrent vulvovaginal or pelvic pain during penetration; 3) marked and persistent or recurrent fear or anxiety about vulvovaginal or pelvic pain in anticipation of, during, or as a result of penetration. The symptoms are recurrent during sexual interactions involving or potentially involving penetration, despite adequate sexual desire and stimulation, and are not entirely attributable to a medical condition that adversely affects the pelvic area and results in genital and/or penetrative pain, or to a mental disorder, or to insufficient vaginal lubrication or post-menopausal/ age-related changes, and are associated with clinically significant distress.

In one embodiment the sexual pain-penetration disorder is lifelong sexual pain-penetration disorder or acquired sexual pain-penetration disorder. In one embodiment, the sexual pain-penetration disorder is generalised sexual pain-penetration disorder or situational sexual pain-penetration disorder. In one embodiment, the sexual pain-penetration disorder is unspecified sexual pain-penetration disorder. Lifelong, generalised sexual pain-penetration disorder is characterised by the subject having always experienced genito-pelvic pain or penetration disorder from the time of initiation of relevant sexual activity and the desired response is currently absent or diminished in all circumstances, including masturbation. Lifelong, situational sexual pain-penetration disorder is characterised by the subject having always experienced genito-pelvic pain or penetration disorder from the time of initiation of relevant sexual activity and the desired response is currently absent or diminished in some circumstances, with some partners, or in response to some stimuli, but not in other situations. Acquired, generalised sexual pain-penetration disorder is characterised by an onset of genito-pelvic pain or penetration disorder following a period of time during which the person did not experience it and the desired response is currently absent or diminished in all circumstances, including masturbation. Acquired, situational sexual pain-penetration disorder is characterised by an onset of genito-pelvic pain or penetration disorder following a period of time during which the person did not experience it and the desired response is currently absent or diminished in some circumstances, with some partners, or in response to some stimuli, but not in other situations. In one embodiment, the sexual pain-penetration disorder is lifelong, generalised sexual pain-penetration disorder. In one embodiment, the sexual pain-penetration disorder is lifelong, situational sexual pain-penetration disorder. In one embodiment, the sexual pain-penetration disorder is acquired, generalised sexual pain-penetration disorder. In one embodiment, the sexual pain-penetration disorder is acquired, situational sexual pain-penetration disorder.

In one embodiment, the sexual pain-penetration disorder is characterised by:
(i) marked or persistent or recurrent difficulties with penetration, including due to involuntary tightening or tautness of the pelvis floor muscles during attempted penetration,
(ii) marked and persistent or recurrent vulvovaginal or pelvic pain during penetration, and/or
(iii) marked and persistent or recurrent fear or anxiety about vulvovaginal or pelvic pain in anticipation of, during, or as a result of penetration.

In one embodiment, the sexual pain-penetration disorder has symptoms that:
(i) are recurrent during sexual interactions involving or potentially involving penetration, despite adequate sexual desire and stimulation,
(ii) are not entirely attributable to a medical condition that adversely affects the pelvic area and results in genital and/or penetrative pain or to a mental disorder,
(iii) are not entirely attributable to insufficient vaginal lubrication or postmenopausal or age-related changes, and
(iv) are associated with clinically significant distress.

In one embodiment, the pain is vulvar pain. Vulvar pain may be caused by specific disorders, such as infectious causes, inflammatory causes, neoplastic causes, neurologic causes, injury or trauma, iatrogenic causes or hormonal deficiencies. In one embodiment, the vulvar pain has neurologic causes, for example the vulvar pain is caused by postherpetic neuralgia, nerve compression, nerve injury or neuroma.

In one embodiment, the pain is vulvodynia, or persistent vulvar pain. Vulvodynia is a persistent, unexplained pain in the vulva, which is the female genital area including the skin surrounding the opening of the vagina. In one embodiment, the vulvodynia is localized vulvodynia, such as vestibulodynia, clitorodynia; or generalized vulvodynia. In one embodiment, the vulvodynia is mixed vulvodynia having both localized and generalized vulvodynia components. The vulvodynia may be associated, or comorbid, with other conditions.

In one embodiment, the pain is pain associated with genitourinary syndrome of menopause (GSM). GSM is a collection of symptoms and signs caused by changes to the labia majora/minora, clitoris, vestibule/introitus, vagina, urethra, and bladder that occur in menopausal patients. The term geniroturinary syndrome of menopause has been used to replace the terms vaignal atrophy or vulvovaginal atrophy, urogenital atrophy, or atrophic vaginitis. The symptoms of GSM include, but are not limited to, genital symptoms of dryness, burning, and irritation; sexual symptoms of lack of lubrication, discomfort or pain, and impaired function; and urinary symptoms of urgency, dysuria, and recurrent urinary tract infections. In one embodiment, the pain is associated with genitourinary syndrome of menopause or pain associated with vaginal atrophy, vulvovaginal atrophy, urogenital atrophy, or atrophic vaginitis. In one embodiment, GSM or vaginal atrophy is due to hypoestrogenic causes.

In one embodiment, the compound is able to reduce sexual pain.

One embodiment provides a compound of formula (I) as described herein, for use in the treatment, prevention or alleviation of pain, such as a type of pain as described herein above, wherein the compound is administered in an amount form 0.5 to 10 mg per individual dose, such as from 1 to 10 mg, preferably from 5 to 10 mg per individual dose.

One embodiment provides a compound of formula (I) as described herein, for use in the treatment, prevention or alleviation of neuropathic pain, such as a type of neuropathic pain as described herein above, wherein the compound is administered in an amount form 0.5 to 10 mg per individual dose, such as from 1 to 10 mg, preferably from 5 to 10 mg per individual dose.

One embodiment provides a compound of formula (I) as described herein, for use in the treatment, prevention or alleviation of sexual pain, such as a type of sexual pain as described herein above, wherein the compound is administered in an amount form 0.5 to 10 mg per individual dose, such as from 1 to 10 mg, preferably from 5 to 10 mg per individual dose.

One embodiment provides a compound of formula (I) as described herein, for use in the treatment, prevention or alleviation of trigeminal neuralgia, wherein the compound is administered in an amount form 0.5 to 10 mg per individual dose, such as from 1 to 10 mg, preferably from 5 to 10 mg per individual dose.

One embodiment provides a compound of formula (I) as described herein, for use in the treatment, prevention or alleviation of vulvodynia or progressive vulvodynia or vulvar pain as described herein, wherein the compound is administered in an amount form 0.5 to 10 mg per individual dose, such as from 1 to 10 mg, preferably from 5 to 10 mg per individual dose.

In one embodiment, the pain occurs at least for a 1 minute, such as at least for 5 minutes, such as at least for 10 minutes, such as at least for 30 minutes.

In one embodiment, the pain occurs at least for 1 minutes, such as at least for 5 minutes, such as at least for 10 minutes, such as at least for 30 minutes, such at least for 1 hour, such as at least for 2 hours, such as for more than 2 hours.

In one embodiment, the pain occurs or recurs for at least one day, such as for at least 3 days, such as for at least for a week.

In one embodiment, the compound for use as described herein is capable of inducing a pain reduction in a subject of at least 5%, such as at least 10%, such as at least 15%, such as 20%, such as 30%, such as 40%, such as 50%, such as 60%, such as 70% or higher pain reduction in a subject.

In one embodiment, the compound for use as describe herein is capable of reducing pain sensitivity.

The pain response or reduction can be assessed by subjective pain measurements, such as subjective measurements performed on a visual analogue scale (VAS). For example having a 100 mm line with 0 representing "no pain" and 100 representing "worse pain imaginable". Subjects can be asked to mark the VAS using a single vertical stroke at the point they consider to appropriately reflect their level of pain.

The pain response or pain reduction can also be assessed by a numeric rating scale (NRS). For example, by using an 11-point numeric rating scale ranging from 0 ("no pain") to 10 ("worst possible pain") and asking the subject to rate their pain.

Pain response or reduction may also be measured through other subjective measurements, such as by rating response to a questionnaire with a numeric scale. For example, sexual pain may be evaluated with patient reported outcome (PRO) questionnaires, with questions related to different aspect of sexual pain and evaluating the response before, after and/or during treatment. The same approach can be used with other types of pain. For example, in females sexual pain can be measured using the female sexual dysfunction index (FSFI) pain domain questions.

In one embodiment, the pain response or reduction upon treatment as described herein is measured with a subjective measurement as described above, such as a visual analogue scale, a numeric rating scale or a patient reported outcome.

In one embodiment, the compound for use as described herein is capable of improving sensory parameters, such as detection and pain thresholds. For example, the compound for use as described herein may improve mechanical pain threshold and/or detection, or thermal detection and/or thermal pain thresholds, such as cold pain thresholds or heat pain thersholds, mechanical pain sensitivity, wind-up ratio, and pressure pain threshold.

In one embodiment, the compound administered as described herein is capable of improving mechanical pain threshold. In one embodiment, the compound administered as describe herein is capable of improving pressure pain detection. In one embodiment, the compound administered as described herein is capable of improving mechanical pain sensitivity. In one embodiment, the compound administered as described herein is capable of improving thermal pain threshold. In one embodiment, the compound administered as described herein is capable of improving heat pain threshold. In one embodiment, the compound administered as described herein is capable of improving cold pain threshold. In one embodiment, the compound administered as described herein is capable of improving thermal pain sensitivity, such as heat or cold pain sensitivity. In one embodiment, the compound administered as described herein is capable of improving pressure pain threshold. In one embodiment, the compound administered as described herein is capable of improving pressure pain sensitivity.

In one embodiment, the compound for use as described herein is capable of improving the functionality in a subject having pain, such as an improvement of sleep quality, such as an improvement of quality of life, such as an improvement of preservation of social activity and relationships, or such as maintained or recovered ability to work or perform activities.

In one embodiment, the improvement in functionality is an improvement of sleep quality. In one embodiment, the improvement in functionality is an improvement of quality of life. In one embodiment, the improvement in functionality is an improvement of is a maintained or recovered ability to work or perform activities.

As shown in the examples, doses of 5 and 10 mg of the compound produce beneficial effects in models of neuropathic pain whilst being associated with lower side effects than higher doses. These results highlight the advantage of establishing a chronic dosage regime with individual doses of or below 10 mg such that a steady state plasma levels of IP2015 can lead to positive therapeutic outcomes without detrimental side effects, for example chronic dosage regime with individual doses of 0.5 to 10 mg, such as individual doses of 0.5 to 5 mg, such as 0.5 mg to 1 mg, 1 mg to 2 mg, 2 mg to 3 mg, 3 mg to 4 mg, 4 mg to 5 mg per individual dose.

Thus, in one embodiment the compound of formula I is administered in individual doses from 0.5 to 10 mg as described herein, once a day, two times a day, three times a day or four times for a period of time of a week, two weeks, three weeks or four weeks. In one embodiment, the compound is adminiered once a day, two times a day, three times a day or four times a day for a period of a month or more, such as two months, three months, six months or a year.

Thus, in one embodiment the compound of formula I is administered in individual doses from 0.5 to 10 mg as described herein once a week, two times a week, three times a week, four times a week, or five times a week, for a period of time of a week, two weeks, three weeks or four weeks. In one embodiment, the compound is adminiered once a week, two times a week, three times a week, four times a week, or five times a week for a period of a month or more, such as two months, three months, six months or a year.

In one embodiment, the subject is a mammal. In one embodiment, the mammal is a human.

The compound for use according to the present disclosure may be combined with one or more further therapeutic agent(s). In one embodiment, the subject is administered with one or more further therapeutic agent(s) effective for the treatment or prevention of pain. In one embodiment, the one or more further therapeutic agents are selected form the group consisting of: opioid receptor agonists, such as tramadol or tapentadol, methadone, nalbuphine, butorphanol, buprenorphine, oxycodone or morphine; and non-steroidal anti-inflammatory drugs such as COX-2 inhibitors, diclofenac, naproxen, ibuprofen, celecoxib, mefenamic acid, etoricoxib or indomethacin.

In one embodiment, the one or more further therapeutic agent(s) are effective for the treatment of neuropathic pain. In one embodiment, the one or more further therapeutic agent(s) are selected from the group consisting of: gamma-amino butyric acid analogs, such as gabapentin or pregabalin; capsaicinoids, such as capsaicin; dual serotonin-norephrine reuptake inhibitors, such as duloxetine; anticonvulsants, such as carbamazepine or oxacarbazepine; tricyclic antidepressants, such as amitriptyline or nortriptyline; TRPV1 receptor modulators, such as a TRPV1 receptor agonists or a TRPV1 receptor antagonists, neurokinin receptor modulators, such as neurokinin receptor antagonists or neurokinin receptor agonists.

In one embodiment, the administration of the compound of formula I is an oral administration. In one embodiment, the administration of a compound of formula I is parenteral administration, such as cutaneous, mucosal, subcutaneous, intramuscular, intraperitoneal, intravenous or intraarterial injection.

In one embodiment, the compound for use as described herein is formulated in a pharmaceutical composition further comprising a pharmaceutically acceptable diluent, carrier and/or excipient. In one embodiment, the compound is formulated as a solid dosage form, such as a tablet, a capsule, a pill, granules or a powder.

In one embodiment, the compound according to formula (I) and the one or more further therapeutic agent(s) as described herein are administered in the same formulation.

### Medical use

The inventors have shown that administration of a compound of formula I as described herein is useful for the treatment of neuropathic pain. Thus, the present disclosure provides for the use of a compound of formula I as described in the section "Compounds for use".

### Examples

### Example 1: In vitro potency

### Materials and Methods

Compounds were tested for their ability to inhibit the reuptake of the monoamine neurotransmitters dopamine (DA), noradrenaline (NA), and serotonin (5-HT) in synaptosomes as described in WO 97/16451 (NeuroSearch A/S). In addition, the compounds were tested in human epithelial cells expressing human transporters for 5-HT (SERT), dopamine (DAT), and norepinephrine (NET).

### Results

The test values are given as IC₅₀ (the concentration (µM) of the test substance which inhibits the specific binding of ³H-DA, ³H-NA, or ³H-5-HT by 50 %). See Table A.

**Table A: Test results obtained by testing exo-7-[(8-azabicyclo[3.2.1]octan-3-yl)oxy]-3-methoxy-chromen-2-one.**

| 5-HT-uptake IC₅₀ (µM) | DA-uptake IC₅₀ (µM) | NA-uptake IC₅₀ (µM) |
|---|---|---|
| 0.0029 | 0.07 | 0.0038 |

The concentrations causing 50% inhibiton at the 5-hydroxytryptamine transporter (5-HTT or SERT), norepinephrine transporter (NET) and dopamine transporter (DAT) in human epithelial cells are shown in Table B.

**Table B: Concentrations of IP2015 causing 50% inhibition (IC₅₀, nM) for each human transporter 5-HTT, NET or DAT in stable cell lines. The results are the average of two-replicates per experiment (n=2).**

| 5-HTT | NET | DAT |
|---|---|---|
| 0.2975^{a} | 95.03 | 1.444 |

| | | |
|---|---|---|
| ^{a} Maximal inhibition at 5-HTT for IP2015 was 80%. | | |

### Example 2: Microdialysis studies in mice brain

### Materials and methods

In a small subset of mice, microdialysis was performed with a probe placed in either the striatum or cortex by use of stereotaxis. After subcutaneous injection of IP2015 10 mg/kg, perfusion liquid was sampled, and the amines were measured by high pressure liquid chromatography and standardised to an internal sample.

### Results

The results displayed in Figure 1 show that dopamine was increased in cortex and less in striatum compared to NA and 5-HT.

### Conclusion

These findings support that IP2015 is a monoamine reuptake inhibitor with preference for DAT. The different increases in amines in the 2 locations agree with the in vivo binding profile of IP2015 and with the known expression of DAT, NAT, and SERT.

### Example 3: Effects of IP2015 in the rat chronic constriction injury model of neuropathic pain

Chronic pain, especially neuropathic pain, is characterized by spontaneous pain and sensory abnormalities. Multiple mechanisms including ectopic activity in primary afferents, induction of central sensitization in response to increased primary afferent drive and spinal disinhibition underlie these signs and symptoms after injury.

The chronic constriction injury (CCI) model of neuropathic pain is a well characterized preclinical model that is typically used as a primary behavioural screen for assaying the antinociceptive activity of compounds in laboratory rodents. After CCI, increased sensitivity to mechanical, heat and cold stimulation of the injured hindpaw occurs and typically lasts for 6-8 weeks thereafter.

The goal of the current study was to assess the antinociceptive effects of the triple monoamine reuptake inhibitor IP2015 in this rodent model of neuropathic pain.

### Materials and methods

IP2015 was dissolved in 15 % hydroxypropyl-b-cyclodextrin (Sigma) in 50 mM phosphate buffer. All treatments were administered s.c. in a dosing volume of 2 ml/kg 60 min prior to behavioural assessment.

Adult male Sprague-Dawley rats (Harlan Scandinavia, Alleroed, Denmark) were used. They were housed in Macrolon III cages (20 x 14 x 18 cm or 20 x 40 x18 cm; in groups of 2-5 per cage according to weight) containing wood-chip bedding material (3 x 1 x 4 mm). The environment was temperature (20 ± 2°C) and humidity (55 ± 15%) controlled and consisted of a light-dark cycle of 13:11 h (lights on at 06.00 h and off at 19.00 h). Food (Altromin^{®}) and water were available *ad libitum.* The rats were allowed to habituate to the housing facilities for at least one week prior to being randomly assigned to behavioural experiments. All experiments were performed according to the Ethical Guidelines of the International Association for the Study of Pain (Zimmermann, 1983) and the Danish Committee for Experiments on Animals.

### Study design

All drug testing in CCI rats was performed blinded by the observer. A 2-3 day drug washout period was allowed, and the studies were conducted between 4-5 weeks after surgery. CCI rats used in this study had also been previously enrolled in similar experiments assessing the efficacy of other compound(s). Only those animals with distinct neuropathic behavior (von Frey response <4 g, pin prick response >10 s) were included in the study. The experimental groups are shown in Table C.

**Table C: Experimental groups**

| Group | Model | Treatment | Dose (mg/kg) | Dose volume | Route | n |
|---|---|---|---|---|---|---|
| 1 | CCI | Veh | 0 | 2 ml/kg | s.c. | 8 |
| 2 | CCI | IP2015 | 3 | 2 ml/kg | s.c. | 8 |
| 3 | CCI | IP2015 | 10 | 2 ml/kg | s.c. | 8 |
| 4 | CCI | IP2015 | 30 | 2 ml/kg | s.c. | 2 |

### Procedures

Chronic constriction injury procedure. A chronic constriction injury (CCI) was performed in rats (body weight 180-220 g at the time of surgery) under isoflurane anaesthesia as described previously (Bennett and Xie, 1988). The sciatic nerve was exposed at the mid-thigh level proximal to the sciatic trifurcation. Four chromic gut ligatures (4/0) (Ethicon, New Brunswick, NJ) were tied loosely around the nerve, 1-2 mm apart, such that the vascular supply was not compromised. The overlying muscle was closed in layers with 4/0 synthetic absorbable surgical suture. The skin was closed and sutured with 4/0 silk thread.

Behavioural testing of nerve-injured rats. Nerve-injured rats were routinely tested for the presence of pain-like behaviours for up to 5 weeks after surgery, according to previously described methods (Munro et al., 2008). For testing of mechanical hypersensitivity individual rats were removed from their home cage and allowed to habituate for 15 min in a 15 x 20 cm white Plexiglas testing cage, placed upon an elevated metal grid allowing access to the plantar surface of the injured hindpaw. The presence of mechanical allodynia was assessed using a series of calibrated von Frey hairs (lower limit=0.065 and upper limit=19.3 g, Stoelting Co, Wood Dale IL), which were applied to the plantar surface of the hindpaw with increasing force until an individual filament used just started to bend. The filament was applied for a period of 1-2 s and was repeated 5 times at 1-2 s intervals. The filament that induced a reflex paw withdrawal in 3 out of 5 applications was considered to represent the threshold level for a response to occur. The presence of mechanical hyperalgesia was determined by pressing the plantar surface of the hindpaw with the point of a safety pin, at an intensity sufficient to produce a reflex withdrawal response in normal unoperated animals, but at an intensity which was insufficient to penetrate the skin. A cut-off time of 15 s was applied to long withdrawals often seen for the nerve-injured paw.

Changes in hindpaw weight bearing were assessed using an incapacitance tester (Linton Instrumentation, U.K.), which incorporates a dual channel scale used to separately assess the weight distributed to each hindpaw of the rat. Normally, uninjured rats distribute their weight evenly between the two hindpaws (50:50). However, after tissue injury the rat preferentially favours the non-injured hindpaw, such that the weight bearing difference can be used as an index of spontaneous nociception. A rat was placed in the supplied Perspex chamber which is designed so that each hindpaw must be placed on separate transducer pads. The testing duration was set to 5 s and the digital read out for each hindpaw was taken as the distributed body weight on each paw (g). Three readings were obtained to ensure that consistent responses were measured. These were averaged for each hindpaw and the weight bearing difference calculated as the difference between the two hindpaws.

### Statistics

Analysis of the data was initially performed using Sigmastat 2.03 (SPSS Inc., Chicago, ILL.) and re-confirmed using GraphPadPrism v8.0.3. All data are presented as mean ± S.E.M. One way analysis of variance (ANOVA) was used to analyse the overall effects of treatments. When the F value was significant this was followed by Dunnett's test. P<0.05 was considered to be statistically significant.

### Study deviations

Rats injected with 10 and especially 30 mg/kg IP2015 appeared to be extremely alert, and possibly displayed signs of anxiogenic behaviour. Due to the nature of the behavioural testing procedures employed which optimally require rats to be calm and relatively sedentary, only 2 rats were injected with 30 mg/kg IP2015. Accordingly, due to the low group *n* this treatment was not included in the overall data analysis.

### Results

Figure 2 shows the results of the present study.

IP2015 produced a small but significant increase in the hindpaw withdrawal threshold to mechanical von Frey stimulation in CCI rats. A more robust dose-dependent reversal of CCI induced weight bearing deficits was obtained after IP2015 administration indicating that IP2015 might be especially effective in treating signs of spontaneous, ongoing pain after neuropathic injury.

Animals in the vehicle group exhibited low thresholds for von Frey stimulation (around 1.1 g). In contrast, animals treated with IP2015 were much less sensitive to stimulation, with the 3 mg IP2015 per kg body weight group exhibiting a paw withdrawal threshold of about 5.7 g, and the 10 mg IP2015 per kg body weight group exhibiting a paw withdrawal threshold of about 3.6 g.

Animals in the vehicle group showed paw withdrawal durations of about 14.9 s. In comparison, animals treated with IP2015 showed shorter paw withdrawal durations, specifically about 12.3 s for the group treated with 3 mg IP2015 per kg body weight, and about 11.1 s for the group treated with 10 mg IP2015 per kg body weight.

Regarding weight bearing, a clear difference between the vehicle groups and the IP2015 groups were also observed. For the vehicle group, the animals showed about 51 % weight bearing difference. In contrast, the 3 mg IP2015 per kg body weight group showed a low difference of about 17 %, whereas the 10 mg IP2015 per kg body weight group showed a yet lower difference of about 11 %.

### Conclusion

IP2015 produced a small but significant increase in the hindpaw withdrawal threshold to mechanical von Frey stimulation in CCI rats. A more robust dose-dependent reversal of CCI-induced weight bearing deficits was obtained after IP2015 administration indicating that IP2015 might be especially effective in treating signs of spontaneous, ongoing pain after neuropathic injury.

### Example 4: Effects of IP2015 in the automated rat formalin test of persistent nociceptive pain

Chronic pain, especially neuropathic pain, is characterized by spontaneous pain and sensory abnormalities. Multiple mechanisms including ectopic activity in primary afferents, induction of central sensitization in response to increased primary afferent drive and spinal disinhibition underlie these signs and symptoms after injury.

The formalin test is used as a primary behavioural screen for assaying the antinociceptive activity of compounds in laboratory rodents. After hindpaw formalin injection, nociceptive behaviours are expressed in a biphasic pattern separated by a period of quiescence called interphase. From a purely construct standpoint, the component phases display reasonable overlap with various mechanistic aspects of clinical neuropathic pain, thereby supporting its routine use as a primary behavioural screen for assaying the antinociceptive activity of novel compounds.

The goal of the current study was to assess the antinociceptive effects of the triple monoamine reuptake inhibitor IP2015 in this animal model.

### Materials and methods

IP2015 was dissolved in 15% hydroxypropyl-b-cyclodextrin (Sigma) in 50 mM phosphate buffer. All treatments were administered s.c. in a dosing volume of 2 ml/kg 60 min prior to formalin injection.

Adult male Sprague-Dawley rats (Harlan Scandinavia, Alleroed, Denmark) were used. They were housed in Macrolon III cages (20 x 14 x 18 cm or 20 x 40 x18 cm; in groups of 2-5 per cage according to weight) containing wood-chip bedding material (3 x 1 x 4 mm). The environment was temperature (20 ± 2°C) and humidity (55 ± 15%) controlled and consisted of a light-dark cycle of 13:11 h (lights on at 06.00 h and off at 19.00 h). Food (Altromin^{®}) and water were available *ad libitum.* The rats were allowed to habituate to the housing facilities for at least one week prior to being randomly assigned to behavioural experiments. At the end of each experiment rats were euthanized by cervical dislocation. All experiments were performed according to the Ethical Guidelines of the International Association for the Study of Pain (Zimmermann, 1983) and the Danish Committee for Experiments on Animals.

### Study design

As highlighted in the Procedures section below 4 rats were included in each automated testing session. 4 individual treatments were assessed during the session and treatments were rotated to minimise bias associated with a particular testing chamber. The experimental groups are shown in Table D.

**Table D: Experimental groups**

| **Group** | **Model** | **Treatment** | **Dose (mg/kg)** | **Dose volume** | **Route** | **n** |
|---|---|---|---|---|---|---|
| 1 | Form | Veh | 0 | 2 ml/kg | s.c. | 8 |
| 2 | Form | IP2015 | 1 | 2 ml/kg | s.c. | 4 |
| 3 | Form | IP2015 | 3 | 2 ml/kg | s.c. | 8 |
| 4 | Form | IP2015 | 10 | 2 ml/kg | s.c. | 8 |
| 5 | Form | IP2015 | 30 | 2 ml/kg | s.c. | 8 |

### Procedures

Assessment of formalin-induced flinching behaviour in normal, uninjured rats (body weight 180-220 g) was made with the use of an Automated Nociception Analyser (University of California, San Diego, CA; Yaksh et al., 2001). Briefly, this involved placing a small C-shaped metal band (10 mm wide x 27 mm long) around the hindpaw of the rat to be tested. Each rat (four rats were included in each testing session) was administered drug or vehicle according to the experimental paradigm being followed, and then placed in a cylindrical acrylic observation chamber (diameter 30.5 cm and height 15 cm). Individual rats were then gently restrained and formalin (5% in saline, 50 ml, s.c.) was injected into the dorsal surface of the hindpaw using a 27G needle. They were then returned to their separate observation chambers, each of which were in turn situated upon an enclosed detection device consisting of two electromagnetic coils designed to produce an electromagnetic field in which movement of the metal band could be detected. The analogue signal was then digitized and a software algorithm applied to enable discrimination of flinching behaviour from other paw movements prior to binning into 1 min sampling intervals. In an initial formalin concentration response study, five phases of nociceptive behaviour were identified and scored according to Yaksh et al., (2001); first phase (P1=0-5 min), interphase (Int=6-15 min), early second phase (P2A=16-40 min), late second phase (P2B=41-60 min) and entire second phase (P2A+P2B=16-60 min). Three of these phases were subsequently chosen for drug administration experiments; first phase (P1=0-5 min), interphase (Int=6-15 min) and early second phase, hereafter referred to as second phase (P2=16-40 min), (Munro et al., 2007). Raw data from the 1 min sampling intervalues was summed for each phase to obtain the total number of flinches occurring during that period.

### Statistics

Analysis of the data was initially performed using Sigmastat 2.03 (SPSS Inc., Chicago, ILL.) and re-confirmed using GraphPadPrism v8.0.3. All data are presented as mean ± S.E.M. One way analysis of variance (ANOVA) was used to analyse the overall effects of treatments. When the *F* value was significant this was followed by Dunnett's test. P<0.05 was considered to be statistically significant.

### Study deviations

All rats injected with either 10 or 30 mg/kg IP2015 displayed spontaneous erections and/or ejaculations. This did not prevent their enrolment into the formalin test.

The automated testing procedure used here enables data to be analysed off-line after each single testing session. Importantly, this permits either higher or lower doses of a compound to be added before a study is finalized. Typically, other treatments would be tested together with an additional treatment group to help minimize bias within a single testing session. Accordingly, when it became apparent that a minimal effective dose of IP2015 was likely to be <3 mg/kg especially during interphase and 2^{nd} phase, an extra 4 rats were injected with a 1 mg/kg dose of IP2015.

### Results

Figure 3 shows the results of the present study. Figure 3 (a) shows flinches per minute in vehicle group and animals treated with 1, 3, 10, or 30 mg IP2015 per kg body weight. IP2015 reduced the prevalence of flinches in both P1, interphase, and P2 in a dose-dependent manner. Figure 3 (b) shows total flinches in P1, interphase, and P2 in a dose-dependent manner.

### Conclusion

IP2015 produced a robust dose-dependent inhibition of spontaneous nociceptive behaviours throughout the duration of the rat formalin test. This inhibition was especially prevalent during interphase which is a period associated with recruitment of descending inhibitory and spinal inhibitory control mechanisms

### Example 5: A Randomised, Double-Blind, Placebo-Controlled Study to Investigate the Pharmacodynamic Effects of IP2015 in Healthy Male Subjects Using the Intradermal Capsaicin Model

The primary objective of the study was to determine the pharmacodynamic (PD) effects of IP2015 in the intradermal (ID) capsaicin model in healthy male subjects.

### Materials and methods

**Study design.** This was a Phase I, randomized, double-blind, placebo-controlled, 4-way crossover study to investigate the pharmacodynamic effects, safety, tolerability, and pharmacokinetic/ pharmacodynamic correlation of two single oral dose levels of IP2015 compared to 300 mg pregabalin and placebo in healthy male subjects using the intradermal capsaicin model.

**Participants.** The volunteers were males aged 18 to 55 years with a body mass index (BMI) of 18 to 30 kg/m2. They were healthy as determined by a responsible physician, based on a medical evaluation including medical history, physical examination, concomitant medication, vital signs, 12-lead ECG, and clinical laboratory evaluations. The participants must have been in good general health with a skin type compatible with the measures and without significant skin allergies, pigmentary disorders or any active dermatological conditions. Moreover, they must have been able to tolerate the capsaicin injection during screening and demonstrated positive hyperalgesia as defined by an area of hyperalgesia ≥15 cm2 15 minutes after ID administration of 100 µg capsaicin at the additional screening visit at least 7 days before the first dosing. Only non-smokers or participants who did not smoke more than 5 cigarettes per day (or equivalent e-cigarette use) were accepted. The participants provided written informed consent, which included compliance with the requirements and restrictions listed in the consent form, including the use of a condom during the trial and for 3 months after their final dose of trial medication, if their partner was a woman of childbearing potential.

**Randomisation.** A randomisation scheme was produced by MAC Clinical Research. After informed consent was obtained, subjects were allocated a unique Screening number. Only subjects who complied with all the inclusion criteria, and none of the exclusion criteria, were randomised onto the study. The subjects were assigned a randomisation number in the order of recruitment. All screened subjects were identifiable throughout the study.

A permuted blocks randomisation schedule was produced by an unblinded MAC statistician, using SAS PROC PLAN. Subjects were randomly allocated to one of the four treatment sequences in a ratio of 1:1:1:1. The treatment sequences were allocated using a Latin square design. Each of the four treatments were administered across the four treatment periods according to the allocated treatment sequence. Treatment sequence remained blinded until database lock.

**Intervention.** This study was designed to investigate the PD effects, safety, tolerability, and PK/PD correlation of two single oral dose levels of IP2015 compared to 300 mg pregabalin and placebo in healthy male subjects using the ID capsaicin model.

A crossover design was utilized to permit a within-subject comparative assessment of the PD, safety, and PK of two single-dose levels of IP2015 compared to 300 mg pregabalin and placebo in healthy male subjects. A minimum washout period of 5 days was selected based on the half-life (t1/2) of IP2015 and pregabalin, which should have been sufficient to ensure clearance of the drug.

Placebo was included in the study to permit comparative assessment of the safety and tolerability of IP2015 (5 or 10 mg) and pregabalin and to evaluate the balance of benefit and risk of IP2015.

A double-dummy approach was used in this study. Double dummy is a technique for retaining the blind when administering supplies in a clinical trial when two possible treatments cannot be made identical. Supplies were prepared for IP2015 (active and matched placebo solutions) and for pregabalin (active and placebo capsules). Subjects received a combination of two sets of treatments (IP2015 or placebo solution and pregabalin or placebo capsule) in each treatment period to administer the appropriate randomized treatment to the subject for that period.

**Clinical and laboratory data.** Prior to and 15, 30, 60, 90 and 120 minutes after administration of capsaicin, measurements of pain, hyperalgesia, allodynia and AF were performed. At 2 hours post-capsaicin injection, subjects completed a quantitative sensory testing (QST) battery at two sites on the volar surface of each forearm. One site was the most recent capsaicin injection site, and the other was at an equivalent point on the opposite forearm. All subjects were to be discharged from the CRU at 8 hours post IP2015/placebo solution administration, if the Investigator deemed it safe to do so following the collection of safety assessments, PK blood samples and pain measurements at multiple time points. There was a minimum washout period of 5 days between treatment periods.

***Study Drug, Dose and Mode of Administration:*** IP2015 was provided as a powder in bottles for oral solution. A 5% HPβCD solution was used to dissolve IP2015. Reconstitution was performed by a Pharmacist at the clinical site prior to dosing.

In order to maintain the blind, a double dummy approach was used. In each treatment period, subjects received a solution (containing either 5 mg IP2015, 10 mg IP2015, or placebo) and a capsule (300 mg pregabalin or placebo). The treatment combinations were:
- 5 mg IP2015 solution and placebo capsule
- 10 mg IP2015 solution and placebo capsule
- Placebo solution and 300 mg pregabalin capsule
- Placebo solution and placebo capsule

Subjects were randomised to receive a single administration of a treatment combination during each treatment period. All subjects received each treatment combination once only. As the capsaicin injection was to be administered at the approximate tₘₐₓ for IP2015 and pregabalin, the IP2015 or placebo solution was given 3 hours prior to capsaicin injection, and pregabalin or placebo capsule was given 1 hour prior to capsaicin injection.

***Evaluation methods:*** Pharmacodynamics was assessed through pain measurements (response to ID injection of capsaicin, measurement of pain using a visual analogue scale, area and pain score of hyperalgesia, area and pain score of allodynia, and AF) and the ID capsaicin model. Subjects also completed a QST battery at two sites on the volar surface of either forearm. The QST battery included the following tests: thermal detection and pain thresholds, mechanical detection and pain thresholds, stimulus/response functions, wind-up ratio and pressure pain threshold.

Safety was assessed through AE reporting, 12-lead ECG, vital signs, physical examinations and clinical laboratory evaluations. Pharmacokinetics was assessed by blood sampling.

For measurement of pain, a visual analogue scale (VAS) was used. The VAS consisted of a 100 mm line, with 0 representing "no pain" and 100 representing "worst pain imaginable". Subjects were asked to mark the VAS using a single vertical stroke at the point they considered to appropriately reflect their level of pain from the injection of capsaicin (not general pain). A new VAS was provided for each time point and subjects were not allowed to see their previous VAS responses. The VAS was scored by measuring from the left-hand end of the scale to the point where the subject had marked the line, and the distance in mm was recorded.

Response to pain was also assessed by a numeric rating scale (NRS). For example, by using an 11-point numeric rating scale ranging from 0 ("no pain") to 10 ("worst possible pain") and asking the subject to rate their pain. A NRS scale of 0 to 100 was also used in some assesments.

The thermal tests were performed using a thermode probe (Medoc). Cold and warm detection thresholds were measured first, then cold pain and heat pain thresholds were determined. The mean threshold temperature of 3 consecutive measurements was calculated. All thresholds were obtained with ramped stimuli (1°C/sec) that were terminated when the subject pressed a button. For thermal detection thresholds the ramp back to baseline was 1°C/sec, while for thermal pain thresholds this ramp was chosen at maximum device capacity resulting in approximately a nominal 5°C/sec.

The mechanical detection threshold was measured with a standardised set of modified von Frey nylons that exerted forces upon bending between 0.25 and 512 mN graded by a factor of 2 (1 to 2 seconds contact time). The contact area of the von Frey nylons with the skin was of uniform size and shape (rounded tip, 0.5 mm in diameter) to avoid sharp edges that would have facilitated nociceptor activation. Using the "method of limits", 5 threshold determinations were made, each with a series of ascending and descending stimulus intensities. The final threshold was the geometric mean of these 5 series.

The mechanical pain threshold was measured using custom-made weighted pinprick stimuli as a set of 7 pinprick mechanical stimulators with fixed stimulus intensities (flat contact area of 0.2 mm diameter) that exerted forces of 8, 16, 32, 64, 128, 256, and 512 mN. The stimulators were applied at a rate of 2 seconds on, 2 seconds off in an ascending order until the first percept of sharpness was reached. The final threshold was the geometric mean of 5 series of ascending and descending stimuli. This test was designed to detect pinprick hypoalgesia.

Wind-up ratio: In this test, the perceived intensity of a single pinprick stimulus (256 mN pinprick, when tested over arm) was compared with that of a series of 10 repetitive pinprick stimuli of the same physical intensity (1 per second applied within an area of 1 cm2). The subject was asked to give a pain rating representing the single stimulus, and the estimated mean over the whole series of 10 stimuli using a '0 to 100' numerical rating scale. The whole procedure was repeated 5 times. Wind-up ratio was calculated as the mean rating of the 5 series divided by the mean rating of the 5 single stimuli. Wind-up is a frequency dependent increase in excitability of spinal cord neurons that reaches a plateau after about 5 stimuli, the perceptual correlate of which was described by this ratio.

The pressure pain threshold test was performed with a pressure gauge device with a probe area of 1 cm2 (probe diameter of 1.1 cm) that exerted forces up to 20 kg/cm2 corresponding to approximately 2000 kPa. The pressure pain threshold was determined with three series of ascending stimulus intensities, each applied as a slowly increasing ramp of 50 kPa/second (approximately 0.5 kg/cm² second).

***Statistical methods:*** The primary PD endpoint, the area of brush-evoked hyperalgesia (cm²), was derived from the ID capsaicin model results recorded in the electronic case report form (eCRF), at each timepoint and treatment period/visit.

The following secondary PD endpoints were also derived from the ID capsaicin model in the eCRF:
- Subjective rating of pain (mm)
- Pain score of hyperalgesia, using a numeric rating scale (NRS)
- Area of brush-evoked allodynia (cm2)
- Pain score of brush-evoked allodynia, using an NRS
- AF from the ID capsaicin (cm2)

Summaries of whether the ID capsaicin response was measured were listed by treatment, timepoint and treatment period/visit. Summary statistics were presented, including arithmetic mean, standard deviation (SD), minimum, maximum and median values by treatment, timepoint and treatment period/visit. Effects of IP2015 on the ID capsaicin model endpoint values were analysed with mixed model repeated measures (MMRM).

Other secondary pharmacodynamic (PD) endpoints related to the change in QST battery assessments compared to placebo. Summary statistics for both the test and control area, as well as the change between the two, were given, including arithmetic mean, SD, minimum, maximum, and median values, and coefficient of variation (CV) by overall for each treatment and treatment by visit. Additional exploratory statistical analysis was performed on the QST battery assessments. The effects of IP2015 on the QST endpoint values were analysed using MMRM.

### Results

***Subjective rating of pain:*** Administration of IP2015 had positive effect in the subjective rating of pain. The adjusted mean subjective rating of pain (mm) was lower for 5 mg IP2015, 10 mg IP2015, and 300 mg pregabalin compared to placebo at all post-capsaicin injection time points (Table 1, Figure 4A). Thus, IP2015 decreased dose-dependently the average rating, and the differences was most pronounced for 10 mg IP2015 treatment 15, 30, and 60 min post capsaicin treatment, where IP2015 compared to placebo decreased the rating 7.2 mm, 8.3 mm, and 7.5 mm. Pregabalin, compared to placebo, decreased the rating by 1.5 mm, 3.8 mm, and 4.4 mm, respectively, 15, 30, and 60 min post capsaicin injection.

**Table 1: Subjective Rating of Pain (mm) by MMRM Analysis**

| **Timepoint** | **Statistic** | **5 mg IP2015 (N=24)** | **10 mg IP2015 (N=24)** | **300 mg Pregabalin Capsule (N=24)** | **Placebo Capsule (N=25)** |
|---|---|---|---|---|---|
| Overall [a] | Adjusted Mean [b] | 15.6 | 11.7 | 14.4 | 17.1 |
| | Standard Error | 2.24 | 2.24 | 2.24 | 2.20 |
| | 95% CI | 11.15, 20.07 | 7.20, 16.12 | 9.90, 18.82 | 12.73, 21.47 |

| | | | | | |
|---|---|---|---|---|---|
| Abbreviations: CI - Confidence interval; MMRM - mixed model repeated measures; N - the number of subjects who received the stated treatment. [a] Overall is inclusive of all timepoints. [b] Adjusted mean represents the observed value. | | | | | |

***Area of hyperalgesia:*** Administration of IP2015 had positive effects on the area of hyperalgesia compared to placebo (Figure 4B). Hyperalgesia was increased after the intradermal injection of capsaicin. It was observed that area of hyperalgesia was smaller for 10 mg IP2015 and 300 mg pregabalin than placebo at all post capsaicin injection timepoints, and smaller for 5 mg IP2015 from 60 minutes post-capsaicin injection. The average decrease in hyperalgesic area across all timepoints compared to placebo was 7.8 cm² and 4 cm², respectively, for pregabalin and 10 mg IP2015. The onset of effect of 5 mg IP2015 was more pronounced 60 min post capsaicin and reached maximum 120 min after capsaicin injection. A similar effect in reduction of hyperalgesia area was observed for both doses of IP2015 and pregabalin at 120 min post-injection.

***Pain score after hyperalgesia:*** Administration of IP2105 had positive effects on the pain score after hyperalgesia (Figure 4C). The adjusted mean pain score of hyperalgesia was lower for 5 mg IP2015, 10 mg IP2015 and 300 mg pregabalin compared to placebo at all post-capsaicin injection timepoints. At 120 minutes post-capsaicin injection, the pain score of hyperalgesia was comparable for 300 mg pregabalin, 5 mg IP2015 and 10 mg IP2015. Pregabalin decreased the pain score at all post-capsaicin time points. In the group treated with 10 mg IP2015, the adjusted mean curve was overlapping with the pain scores for pregabalin (Figure 4C).

***Area of brush-evoked allodynia:*** Administration of IP2015 showed positive effects in the area of brush-evoked allodyinia (Figure 4D). Both doses of IP2015 reduced the mean area of brushed-evoked allodynia across all time points.The adjusted mean area of brush-evoked allodynia was lower for 300 mg pregabalin compared to placebo at all time points post capsaicin injection (Figure 4D). The allodynia area for 5 mg IP2015 was lower compared to the placebo from 30 to 120 minutes post-capsaicin injection and significantly lowered (P=0.049) 120 min after capsaicin injection.

**Pain score of allodynia:** Administration of IP2015 showed positive effects in the pain score of brush-evoked allodyinia. The adjusted mean pain score of allodynia (Table 2) across all timepoints was lower for 5 mg IP2015, 10 mg IP2015 and 300 mg pregabalin compared to placebo. The adjusted mean pain score of allodynia was lower compared to placebo for both doses of IP2015 and 300 mg pregablain at all post-capsaicin injection timepoints (Figure 4E).

**Table 2: Pain Score of Brush-evoked Allodynia Using an NRS by MMRM Analysis**

| **Timepoint** | **Statistic** | **5 mg IP2015 (N=24)** | **10 mg IP2015 (N=24)** | **300 mg Pregabalin Capsule (N=24)** | **Placebo Capsule (N=25)** |
|---|---|---|---|---|---|
| Overall [a] | Adjusted Mean [b] | 1.8 | 1.6 | 1.5 | 2.0 |
| | Standard Error | 0.23 | 0.23 | 0.23 | 0.23 |
| | 95% CI | 1.38, 2.31 | 1.09, 2.02 | 1.06, 1.99 | 1.52, 2.44 |

| | | | | | |
|---|---|---|---|---|---|
| Abbreviations: CI - Confidence interval; MMRM - mixed model repeated measures; N - the number of subjects who received the stated treatment. [a] Overall is inclusive of all timepoints. [b] Adjusted mean represents the observed value. | | | | | |

***Quantitative sensory testing:*** A post-hoc analysis was performed on the QST assessments.

The 10 mg IP2015 treatment performed comparably to pregabalin for mechanical pain threshold and wind-up ratio. For the pressure pain threshold, 10 mg of IP2015 showed the best effects.

Overall increases in warm detection threshold between test and control areas were significant for 10 mg IP2015 (p = 0.013) and close to significance for 300 mg pregabalin (p = 0.063). The treatment with IP2015 showed improved effects compared to pregabalin in thermal sensory limen warm test.

### Safety evaluation:

No deaths, serious adverse effects (SAEs) or treatment-emergent adverse effects (TEAEs) leading to withdrawal occurred during the study. Overall, 20 (80.0%) subjects experienced 67 TEAEs across all treatment groups. Of the 67 TEAEs reported during the study, 63 events reported by 19 (76.0%) subjects were mild in severity and 4 events reported by 3 (12.0%) subjects were moderate. All 4 moderate events were considered to be related to study medication and were reported following administration of 300 mg pregabalin or placebo. The four moderate events comprised of dizziness (2 subjects; 1 subject administered 300 mg pregabalin and 1 subject administered placebo), mood altered (1 subject administered 300 mg pregabalin) and syncope (1 subject administered placebo). All the TEAEs reported following administration of 5 mg IP2015 and 10 mg IP2015 were mild in severity.

TEAEs were reported most frequently by subjects administered 300 mg pregabalin (19 [79.2%] subjects reported 28 events), followed by 10 mg IP2015 (13 [54.2%] subjects reported 19 events). The incidence of TEAEs in subjects administered 5 mg IP2015 (7 [29.2%] reported 10 events) was comparable to that of subjects administered placebo (8 [32.0%] subjects reported 10 events).

### Conclusion

Administration of single doses of IP2015 at doses 5 and 10 mg had positive effects in diverse measurements related to experience neuropathic pain and was well tolerated after transdermal injection of capsaicin in healthy male volunteers.

### Example 6. Determining the safety and tolerability of ascending single doses of IP2015 in healthy male subjects.

The objectives of the study were to determine the safety and tolerability of ascending single doses of IP2015 in healthy male subjects and to determine the single oral dose pharmacokinetics (PK) of IP2015 in healthy male subjects.

### Materials and Methods

**Study design.** The study was a Phase I, randomised, double-blind, placebo-controlled, ascending single oral dose, safety, tolerability, pharmacokinetic (PK) and pharmacodynamics (PD) study of IP2015 in healthy male subjects.

Each subject received one oral dose of IP2015 or matched placebo. The starting dose of IP2015 was 0.01 mg in Cohort 1. The dose level was to be escalated to a maximum of 0.05 mg in Cohort 2 and a maximum of 0.2 mg in Cohort 3. The doses in the remaining five cohorts were to be determined based on the dose escalation criteria. In every cohort, no more than 2 subjects were dosed on the first dosing day (1 active; 1 placebo) such that no more than 1 subject received an active IP2015 dose for the first time at each dose level.

Subjects were required to attend the clinical research unit (CRU) for a screening visit within 28 days prior to dosing. Subjects were admitted to the CRU on Day -1 for collection of baseline safety and pharmacodynamics (PD) assessments and received a dose of IP2015 or placebo on the morning of Day 1, in the fasted state. All subjects remained in the CRU until Day 3 (48 hours postdose) for the collection of safety assessments, PK blood and urine samples, and CNS assessments.

Subjects attended a follow-up visit 5 to 7 days after discharge from the CRU. The duration of participation for each subject was approximately 5 weeks.

**Participants.** The healthy subjects were male, of any ethnic origin, were aged between 18 to 59 years (inclusive), had a BMI of 18 to 32 kg/m² (inclusive), and had a body weight of ≥50 kg. The participants were healthy as determined by a responsible physician, based on medical history, physical examination, concomitant medication, vital signs, 12-lead ECGs and clinical laboratory evaluations. All participants gave a written informed consent, which included compliance with the requirements and restrictions listed in the consent form.

**Randomisation and blinding.**_In Cohorts 1 and 2, 5 subjects in total were randomly assigned to receive either IP2015 (3 subjects) or placebo (2 subjects). Of the first 2 subjects, one was administered placebo and one was administered IP2015. For all other cohorts 3-8, 8 subjects in total were randomly assigned to receive either IP2015 (6 subjects) or placebo (2 subjects). Of the first 2 subjects, one was administered placebo and one was administered IP2015. The randomisation schemes were generated by a statistician using SAS PROC Plan.

The study was conducted in a double-blinded fashion (Investigator and subject/patient blinded). The randomisation list was kept in a secure location until the end of the study.

The planned volume of either IP2015 or placebo was poured into a blinded dosing container and provided to the dosing staff in the CRU.

**Study Drug, Dose and Mode of Administration.** IP2015 and matched placebo were provided as a powder in bottles for oral solution. A 5% hydroxy propyl beta cyclodextrin solution was reconstituted to form placebo, and this was used to dissolve IP2015. Reconstitution was performed by a pharmacist at the clinical site prior to dosing. Until IP2015 was dispensed to the subjects/participants, it was stored at a controlled room temperature of 15 to 25°C.

The IP2015 or matched placebo was administered once on the morning of Day 1 as an oral solution in the fasted state. The dose was taken with 240 mL of water at room temperature. Subjects/participants were fasted overnight prior to dosing until 4 hours postdose. Water was allowed ad libitum except for 1 hour before and 1 hour after dosing.

The doses used in each cohort were as follows:
- Cohort 1 - 0.01 mg
- Cohort 2 - 0.05 mg
- Cohort 3 - 0.2 mg
- Cohort 4 - 0.6 mg
- Cohort 5 - 1.8 mg
- Cohort 6 - 5.4 mg
- Cohort 7 - 16.2 mg
- Cohort 8 - 10 mg

**Evaluation.** The study evaluated the clinical safety data from adverse event (AE) reporting, 12-lead electrocardiogram (ECG), cardiac telemetry, vital signs (standing and supine blood pressure (BP), heart rate (HR), oral temperature and clinical laboratory evaluations (chemistry, haematology, urinalysis) and physical examinations in healthy male subjects.

The study also evaluated plasma PK concentrations and parameters including but not limited to: area under the plasma concentration vs time curve (AUC), from time zero to the last quantifiable concentration (AUC0-t), AUC from zero to infinity (AUC0-∞), observed maximum plasma concentration (Cmax), time to reach maximum plasma concentration (tmax) and terminal elimination half-life (T1/2) in healthy male subjects. Blood samples for the determination of plasma concentrations of IP2015 were collected at predose, and 0.25, 0.5, 1, 1.5, 2, 3, 4, 6, 8, 12, 16, 24, 36 and 48 hours postdose.

CNS assessments (saccadic eye movement and Visual Analogue Scale [VAS] assessments) were carried out at predose, and 1, 2, 4 and 8 hours postdose on Day 1 and prolactin measurements were taken at 24 and 48 hours postdose.

Central nervous system assessments were conducted to determine the potential CNS effects of IP2015, and included the following:
- Saccadic eye movement (sedation test; Cohorts 3 to 8 only in Part A): Saccadic eye movement was measured using a saccadometer, which is a miniaturised, portable device for recording saccadic responses to visual stimuli. Eye movements were measured non-invasively using infra-red reflection, and miniature lasers mounted on the transducer projected small stimuli in front of the subject. A run of 100 saccades were performed at each timepoint.
- Visual Analogue Scale: A VAS was used to assess a series of symptoms (sleepy, hungry, dizzy, nauseated, anxious, irritable) on a scale from 'not at all' to 'extremely'.
- Prolactin levels: Serum prolactin levels were obtained via blood sample and analysed.

The parameters shown in Table E were derived to assess the effect of IP2015.

**Table E. Assessment Parameters**

| **Assessment** | **Parameter** |
|---|---|
| Visual Analogue Scale Assessment | Sleepy |
| | Hungry |
| | Dizzy |
| | Nauseated |
| | Anxious |
| | Irritable |
| Eye Movement Assessment | Latency |
| | Peak Saccadic Velocity |
| | Failed Saccades |

**Statistical methods.** Safety parameters were listed and summarised using descriptive statistics. Pharmacokinetic parameter estimates were calculated using noncompartmental methods. Pharmacokinetic data were listed for each subject/patient and summarised by descriptive statistics.

Dose proportionality was analysed for Part A with a linear regression model using the logarithm of a PK parameter as responsible variable and the logarithm of the dose as the independent variable. The linear regression model can be expressed as:$Log \left({y}_{i}\right) = α + β * log {dose}_{i} + {ε}_{i}$ where α is the intercept, doseᵢ is the actual dose of IP2015 for the ith subject and εᵢ is the (within subject) random error in observing yᵢ.

The above model was applied to the following PK parameters; AUC0-t, AUC0-∞ and Cmax. Based on the linear regression model, the dose proportionality coefficient (slope) and its two-sided 90% confidence interval (CI) were estimated. Dose proportionality was declared if the 90% CI for the slope was completely contained in the following range16: 1+log(0.5)/log(r), 1+log(2)/log(r), where r is the high dose/low dose.

Pharmacodynamic data were listed for each subject, along with summary statistics including arithmetic means, SD, minimum, maximum and median values, by timepoint and dose cohort.

### Results

### Single Dose Pharmacokinetics of IP2015

Plasma concentrations of IP2015 were below the LLOQ for all subjects who received 0.01 mg and 0.05 mg IP2015, and for 3/7 subjects who received 0.2 mg IP2015. The plasma concentration of IP2015 versus time profiles for doses ≥0.6 mg were characterised by a relatively rapid absorption phase. Median tmax was generally similar at each dose, ranging from 2.25 to 5.00 hours postdose, and tmax ranged from 1.00 to 6.00 hours postdose across all dose levels. After reaching Cmax, plasma concentrations of IP2015 appeared to decline in a bi-phasic manner. The mean T½ of IP2015 was generally similar across the 5.4 mg to 16.2 mg doses, with mean values ranging from 23.11 to 26.30 hours. At the lower dose levels of 0.6 mg and 1.8 mg, the mean t½ was shorter (14.49 hours and 16.09 hours, respectively), probably due to the elimination phase not being fully defined at these doses as T1/2 could only be calculated for 1/6 subjects who received 0.6 mg IP2015 and 3/6 subjects who received 1.8 mg IP2015. Half-life could not be calculated for any subject at the 0.2 mg dose level.

Analysis of dose proportionality is presented in Table F. The slope estimate (90% CI) from the regression analysis for Cmax was 1.1389 (1.1005 to 1.1773) for IP2015. The lower limit of the 90% CI was above unity indicating a slightly more than dose-proportional increase for Cmax over the range of 0.2 to 16.2 mg. The slope estimates (90% CI) for AUC0-∞ and AUC0-t were 1.1884 (1.0721 to 1.3048) and 1.5579 (1.4007 to 1.7151), respectively, for IP2015. The lower limit of the 90% CI was above unity for both parameters, and indicated a slightly more than dose-proportional increase in the systemic exposure based on AUC0-∞ and a greater than dose-proportional increase in the systemic exposure based on AUC0-t over the dose range 0.2 to 16.2 mg.

**Table F. Analysis of dose proportionality**

| **Parameter** | **Effect** | **Estimate** | **P-value** | **90% CI** | | **Range** | |
|---|---|---|---|---|---|---|---|
| | | | | **Lower** | **Upper** | **Lower** | **Upper** |
| Cmax (ng/mL) | Intercept | 0.2406 | <0.0001 | 0.1748 | 0.3064 | - | - |
| | Logdose | 1.1389 | <0.0001 | 1.1005 | 1.1773 | 0.7897 | 1.2103 |
| AUC0-t (ng/mL) | Intercept | 2.8503 | <0.0001 | 2.5810 | 3.1179 | - | - |
| | Logdose | 1.5579 | <0.0001 | 1.4007 | 1.7151 | 0.8423 | 1.1577 |
| AUC0-∞ (ng/mL) | Intercept | 3.6152 | <0.0001 | 3.3747 | 3.8558 | - | - |
| | Logdose | 1.1884 | <0.0001 | 1.0721 | 1.3048 | 0.8423 | 1.1577 |

| | | | | | | | |
|---|---|---|---|---|---|---|---|
| Abbreviations: AUC0-∞ - area under the plasma concentration vs time curve from zero to infinity; AUC0-t - area under the plasma concentration vs time curve from time zero to the last quantifiable concentration; CI - confidence interval; Cmax - maximum plasma concentration. | | | | | | | |

### Adverse events:

Overall, 24 (42.1%) subjects experienced 44 treatment-emergent adverse events (TEAEs). The incidence of TEAEs was relatively low over the 0.01 mg to 10 mg dose range. In the 16.2 mg dose group, all 4 subjects reported at least one TEAE. The majority of events were mild in severity and no subjects discontinued due to a TEAE. Of the 40 subjects who received IP2015, 16 subjects experienced a total of 32 TEAEs during the study. Treatment-emergent AEs were reported for all dose groups, with the exceptions of the 0.05 mg and 0.6 mg dose groups. A total of 12 TEAEs were experienced by 8 subjects in the placebo group.

There were no significant treatment- or dose-related trends in the mean or individual subject haematology, serum biochemistry or urinalysis data during the study and no clinically significant findings in the physical examinations performed. There were no significant treatment- or dose-related trends in the mean or individual subject vital sign values over the 0.01 mg to 10 mg dose range. At the 16.2 mg dose level, increases from baseline in standing pulse rate were observed at 2, 3, 4, 6, 8, 10 and 12 hours postdose, respectively, while increases from baseline in supine pulse rate were observed at 6, 8 and 10 hours postdose, respectively. These increases from baseline were the result of 2 subjects who showed sustained tachycardia for several hours.

There were no significant treatment- or dose-related trends in the mean ECG parameters over the 0.01 mg to 10 mg dose range. At the 16.2 mg dose level, increases from baseline in heart rate were observed at 8 and 12 hours postdose, respectively.

### Conclusion

The incidence of TEAEs was relatively low over the 0.01 mg to 10 mg dose range. The incidence of side effects was increased at 16.2 mg compared to the 0.01 mg to 10 mg dose range. The majority of events were mild in severity and no subjects discontinued due to a TEAE.

## Claims

1. A compound of formula (I), 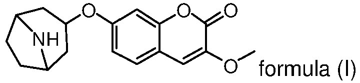 or a pharmaceutically acceptable salt thereof, for use in the treatment, prevention or alleviation of pain in a subject, wherein the compound is administered in an amount of 0.5 mg to 10 mg per individual dose.

2. The compound for use according to any one of the preceding claims, wherein the compound is exo-7-[(8-azabicyclo[3.2.1]octan-3-yl)oxy]-3-methoxy-chromen-2-one hydrochloride.

3. The compound for use according to any one of the preceding claims, wherein the compound of formula I is administered in an amount per individual dose from about 1 mg to about 10 mg, such as from about 3 to about 10 mg per individual dose, such as about 5 mg per individual dose, such as about 10 mg per individual dose.

4. The compound for use according to any one of the preceding claims, wherein the compound is administered in an amount from 5 to 10 mg per individual dose.

5. The compound for use according to any one of the preceding claims wherein the compound is administered once daily.

6. The compound for use according to any one of the preceding claims, wherein the pain is selected from the group consisting of: acute pain, chronic pain, mild pain, moderate or severe pain, postoperative pain, neuropathic pain, central neuropathic pain, pain related to diabetic neuropathy, to postherpetic neuralgia, to peripheral nerve injury, to phantom limb pain, to neurogenic inflammation, to fibromyalgia, to chronic regional pain syndrome; somatic pain, visceral pain or cutaneous pain, pain caused by inflammation or by infection, pain related to arthritis, osteoarthritis, or rheumatoid arthritis; neuronal hyperexcitability disorders, peripheral nerve hyperexcitability, back pain, cancer pain, dental pain, irritable bowel pain, irritable bowel syndrome, post-operative pain, post-mastectomy pain syndrome (PMPS), post-stroke pain, drug-induced neuropathy, complex regional pain syndrome (CRPS), sympathetically maintained pain (SMP), trigeminal neuralgia, myofacial pain, chronic headache, migraine, migraine-related disorders, or tension-type headache.

7. The compound for use according to any one of the preceding claims, wherein the pain is selected from the group consisting of:
(i) chronic or acute primary pain,
(ii) chronic or acute cancer related pain,
(iii) chronic or acute postsurgical pain,
(iv) chronic or acute post-traumatic pain,
(v) chronic or acute secondary musculoskeletal pain,
(vi) chronic or acute secondary visceral pain,
(vii) chronic or acute neuropathic pain, and
(viii) chronic or acute secondary headache or orofacial pain.

8. The compound for use according to any one of the preceding, wherein the pain is neuropathic pain.

9. The compound for use according to any one of the preceding claims, wherein the pain is trigeminal neuralgia.

10. The compound for use according to any one of the preceding claims, wherein the pain is sexual pain disorder, such as vulvar pain, or pain associated with genitourinary syndrome of menopause (GSM), or pain associated with vaginal atrophy, vulvovaginal atrophy, urogenital atrophy, or atrophic vaginitis.

11. The compound for use according to any one of the preceding claims, wherein the pain is vulvodynia or progressive vulvodynia.

12. The compound for use according to any one of the preceding claims, wherein the subject is administered with a further therapeutic agent effective for the treatment of pain; such as wherein the further therapeutic agent is selected from the group consisting of: opioid receptor agonists, such as tramadol or tapentadol, methadone, nalbuphine, butorphanol, oxycodone, or morphine; non-steroidal anti-inflammatory drugs, such as COX-2 inhibitors, diclofenac, naproxen, ibuprofen, celecoxib, mefenamic acid, etoricoxib, or indomethacin; and an agent that is effective for the treatment of neuropathic pain, such as a further therapeutic agent selected from the group consisting of: gamma-amino butyric acid analogs, such as gabapentin, or pregabalin; capsaicinoids, such as capsaicin; dual serotonin-norephrine reuptake inhibitors, such as duloxetine; anticonvulsants, such as carbamazepine, or oxacarbazepine; tricyclic antidespressants, such as amitriptyline, or nortriptyline; transient receptor potential cation channel subfamily V member 1 (TRPV1) receptor modulators, such as a TRPV1 receptor agonist, or a TRPV1 receptor antagonist; and neurokinin receptor modulators, such as neurokinin receptor antagonists or neurokinin receptor agonists.

13. The compound for use according to any one of the preceding claims, wherein the compound is capable of inducing a pain reduction in a subject of at least 5%, such as at least 10%, such as at least 15%, such as 20% pain reduction.

14. The compound for use according to any one of the preceding claims, wherein the administration of the compound is an oral administration.

15. The compound for use according to any one of the preceding claims, wherein the compound is formulated in a pharmaceutical composition further comprising a pharmaceutically acceptable diluent, carrier, and/or excipient; for example wherein the compound is formulated as a solid dosage form, such as a tablet, a capsule, a pill, granules, or a powder.

## Patentansprüche

1. Verbindung der Formel (I), 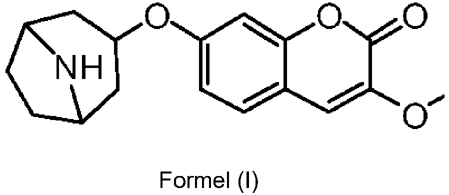 oder ein pharmazeutisch verträgliches Salz davon, zur Verwendung bei der Behandlung, Vorbeugung oder Linderung von Schmerzen in einem Subjekt, wobei die Verbindung in einer Menge von 0,5 mg bis 10 mg pro Einzeldosis verabreicht wird.

2. Verbindung zur Verwendung nach einem der vorhergehenden Ansprüche, wobei es sich bei der Verbindung um exo-7-[(8-Azabicyclo[3.2.1]octan-3-yl)oxy]-3-methoxy-chromen-2-on-Hydrochlorid handelt.

3. Verbindung zur Verwendung nach einem der vorhergehenden Ansprüche, wobei die Verbindung der Formel I in einer Menge pro Einzeldosis von etwa 1 mg bis etwa 10 mg, wie etwa 3 bis etwa 10 mg pro Einzeldosis, wie etwa 5 mg pro Einzeldosis, wie etwa 10 mg pro Einzeldosis, verabreicht wird.

4. Verbindung zur Verwendung nach einem der vorhergehenden Ansprüche, wobei die Verbindung in einer Menge von 5 bis 10 mg pro Einzeldosis verabreicht wird.

5. Verbindung zur Verwendung nach einem der vorhergehenden Ansprüche, wobei die Verbindung einmal täglich verabreicht wird.

6. Verbindung zur Verwendung nach einem der vorhergehenden Ansprüche, wobei der Schmerz ausgewählt ist aus der Gruppe, bestehend aus: akutem Schmerz, chronischem Schmerz, leichtem Schmerz, mäßigem oder starkem Schmerz, postoperativem Schmerz, neuropathischem Schmerz, zentralem neuropathischem Schmerz, Schmerz im Zusammenhang mit diabetischer Neuropathie, mit postherpetischer Neuralgie, mit einer Verletzung eines peripheren Nervs, mit Phantomschmerz, mit neurogener Entzündung, mit Fibromyalgie, mit chronischem regionalem Schmerzsyndrom; somatischem Schmerz, viszeralem Schmerz oder kutanem Schmerz, durch Entzündung oder durch Infektion verursachtem Schmerz, Schmerz im Zusammenhang mit Arthritis, Osteoarthritis oder rheumatoider Arthritis; neuronalen Hypererregbarkeitsstörungen, peripherer Nervenhypererregbarkeit, Rückenschmerz, Krebsschmerz, Zahnschmerz, Reizdarmschmerz, Reizdarmsyndrom, postoperativem Schmerz, Post-Mastektomie-Schmerzsyndrom (PMPS), Schmerz nach Schlaganfall, arzneimittelinduzierter Neuropathie, komplexem regionalem Schmerzsyndrom (CRPS), sympathisch unterhaltenem Schmerz (SMP), Trigeminusneuralgie, myofaszialem Schmerz, chronischem Kopfschmerz, Migräne, migränebedingten Störungen oder Spannungskopfschmerz.

7. Verbindung zur Verwendung nach einem der vorhergehenden Ansprüche, wobei der Schmerz ausgewählt ist aus der Gruppe, bestehend aus:
(i) chronischem oder akutem primärem Schmerz,
(ii) chronischem oder akutem krebsbedingtem Schmerz,
(iii) chronischem oder akutem postchirurgischem Schmerz,
(iv) chronischem oder akutem posttraumatischem Schmerz,
(v) chronischem oder akutem sekundärem muskuloskelettalem Schmerz,
(vi) chronischem oder akutem sekundärem viszeralem Schmerz,
(vii) chronischem oder akutem neuropathischem Schmerz, und
(viii) chronischem oder akutem sekundärem Kopfschmerz oder orofazialem Schmerz.

8. Verbindung zur Verwendung nach einem der vorhergehenden Ansprüche, wobei es sich bei dem Schmerz um neuropathischen Schmerz handelt.

9. Verbindung zur Verwendung nach einem der vorhergehenden Ansprüche, wobei es sich bei dem Schmerz um Trigeminusneuralgie handelt.

10. Verbindung zur Verwendung nach einem der vorhergehenden Ansprüche, wobei es sich bei dem Schmerz um eine sexuelle Schmerzstörung handelt, wie Vulvaschmerz, oder um Schmerz im Zusammenhang mit dem genitourinären Syndrom der Menopause (GSM), oder um Schmerz im Zusammenhang mit vaginaler Atrophie, vulvovaginaler Atrophie, urogenitaler Atrophie oder atrophischer Vaginitis.

11. Verbindung zur Verwendung nach einem der vorhergehenden Ansprüche, wobei es sich bei dem Schmerz um Vulvodynie oder progressive Vulvodynie handelt.

12. Verbindung zur Verwendung nach einem der vorhergehenden Ansprüche, wobei dem Subjekt ein weiteres therapeutisches Mittel verabreicht wird, das für die Behandlung von Schmerzen wirksam ist; wie wobei das weitere therapeutische Mittel ausgewählt ist aus der Gruppe, bestehend aus: Opioidrezeptor-Agonisten, wie Tramadol oder Tapentadol, Methadon, Nalbuphin, Butorphanol, Oxycodon oder Morphin; nichtsteroidalen entzündungshemmenden Arzneimitteln, wie COX-2-Inhibitoren, Diclofenac, Naproxen, Ibuprofen, Celecoxib, Mefenaminsäure, Etoricoxib oder Indometacin; und einem Mittel, das für die Behandlung von neuropathischem Schmerz wirksam ist, wie einem weiteren therapeutischen Mittel ausgewählt aus der Gruppe, bestehend aus: Gamma-Aminobuttersäure-Analoga, wie Gabapentin oder Pregabalin; Capsaicinoiden, wie Capsaicin; dualen Serotonin-Noradrenalin-Wiederaufnahmehemmern, wie Duloxetin; Antikonvulsiva, wie Carbamazepin oder Oxcarbazepin; trizyklischen Antidepressiva, wie Amitriptylin oder Nortriptylin; Modulatoren des Kationenkanals mit transientem Rezeptorpotential, Unterfamilie V, Mitglied 1 (TRPV1), wie einem TRPV1-Rezeptor-Agonisten oder einem TRPV1-Rezeptor-Antagonisten; und Neurokinin-Rezeptor-Modulatoren, wie Neurokinin-Rezeptor-Antagonisten oder Neurokinin-Rezeptor-Agonisten.

13. Verbindung zur Verwendung nach einem der vorhergehenden Ansprüche, wobei die Verbindung in der Lage ist, bei einem Subjekt eine Schmerzreduktion von mindestens 5 %, wie mindestens 10 %, wie mindestens 15 %, wie 20 % Schmerzreduktion, hervorzurufen.

14. Verbindung zur Verwendung nach einem der vorhergehenden Ansprüche, wobei die Verabreichung der Verbindung eine orale Verabreichung ist.

15. Verbindung zur Verwendung nach einem der vorhergehenden Ansprüche, wobei die Verbindung in einer pharmazeutischen Zusammensetzung formuliert ist, die ferner ein pharmazeutisch verträgliches Verdünnungsmittel, einen Träger und/oder einen Hilfsstoff umfasst; zum Beispiel wobei die Verbindung als feste Darreichungsform formuliert ist, wie eine Tablette, eine Kapsel, eine Pille, Granulat oder ein Pulver.

## Revendications

1. Composé de formule (I), 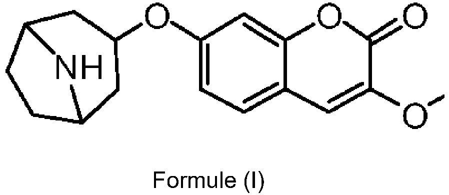 ou un sel pharmaceutiquement acceptable de celui-ci, pour une utilisation dans le traitement, la prévention ou le soulagement de la douleur chez un sujet, le composé étant administré en une quantité de 0,5 mg à 10 mg par dose individuelle.

2. Composé pour une utilisation selon l'une quelconque des revendications précédentes, dans lequel le composé est le chlorhydrate d'exo-7-[(8-azabicyclo[3.2.1]octan-3-yl)oxy]-3-méthoxy-chromén-2-one.

3. Composé pour une utilisation selon l'une quelconque des revendications précédentes, dans lequel le composé de formule I est administré en une quantité par dose individuelle d'environ 1 mg à environ 10 mg, telle que d'environ 3 à environ 10 mg par dose individuelle, telle qu'environ 5 mg par dose individuelle, telle qu'environ 10 mg par dose individuelle.

4. Composé pour une utilisation selon l'une quelconque des revendications précédentes, dans lequel le composé est administré en une quantité de 5 à 10 mg par dose individuelle.

5. Composé pour une utilisation selon l'une quelconque des revendications précédentes, dans lequel le composé est administré une fois par jour.

6. Composé pour une utilisation selon l'une quelconque des revendications précédentes, dans lequel la douleur est sélectionnée dans le groupe constitué par : la douleur aiguë, la douleur chronique, la douleur légère, la douleur modérée ou sévère, la douleur postopératoire, la douleur neuropathique, la douleur neuropathique centrale, la douleur liée à la neuropathie diabétique, à la névralgie post-herpétique, à une lésion d'un nerf périphérique, à la douleur du membre fantôme, à l'inflammation neurogène, à la fibromyalgie, au syndrome douloureux régional chronique ; la douleur somatique, la douleur viscérale ou la douleur cutanée, la douleur causée par une inflammation ou par une infection, la douleur liée à l'arthrite, à l'arthrose ou à la polyarthrite rhumatoïde ; les troubles d'hyperexcitabilité neuronale, l'hyperexcitabilité des nerfs périphériques, la douleur dorsale, la douleur cancéreuse, la douleur dentaire, la douleur du côlon irritable, le syndrome du côlon irritable, la douleur postopératoire, le syndrome douloureux post-mastectomie (PMPS), la douleur post-AVC, la neuropathie d'origine médicamenteuse, le syndrome douloureux régional complexe (SDRC), la douleur entretenue par le système sympathique (SMP), la névralgie du trijumeau, la douleur myofasciale, la céphalée chronique, la migraine, les troubles liés à la migraine, ou la céphalée de tension.

7. Composé pour une utilisation selon l'une quelconque des revendications précédentes, dans lequel la douleur est sélectionnée dans le groupe constitué par :
(i) une douleur primaire chronique ou aiguë,
(ii) une douleur chronique ou aiguë liée au cancer,
(iii) une douleur post-chirurgicale chronique ou aiguë,
(iv) une douleur post-traumatique chronique ou aiguë,
(v) une douleur musculo-squelettique secondaire chronique ou aiguë,
(vi) une douleur viscérale secondaire chronique ou aiguë,
(vii) une douleur neuropathique chronique ou aiguë, et
(viii) une céphalée ou une douleur orofaciale secondaire chronique ou aiguë.

8. Composé pour une utilisation selon l'une quelconque des revendications précédentes, dans lequel la douleur est une douleur neuropathique.

9. Composé pour une utilisation selon l'une quelconque des revendications précédentes, dans lequel la douleur est la névralgie du trijumeau.

10. Composé pour une utilisation selon l'une quelconque des revendications précédentes, dans lequel la douleur est un trouble douloureux sexuel, telle qu'une douleur vulvaire, ou une douleur associée au syndrome génito-urinaire de la ménopause (GSM), ou une douleur associée à l'atrophie vaginale, à l'atrophie vulvovaginale, à l'atrophie urogénitale, ou à la vaginite atrophique.

11. Composé pour une utilisation selon l'une quelconque des revendications précédentes, dans lequel la douleur est la vulvodynie ou la vulvodynie progressive.

12. Composé pour une utilisation selon l'une quelconque des revendications précédentes, dans lequel on administre au sujet un autre agent thérapeutique efficace pour le traitement de la douleur ; par exemple dans lequel l'autre agent thérapeutique est sélectionné dans le groupe constitué par : les agonistes des récepteurs opioïdes, tels que le tramadol ou le tapentadol, la méthadone, la nalbuphine, le butorphanol, l'oxycodone ou la morphine ; les anti-inflammatoires non stéroïdiens, tels que les inhibiteurs de la COX-2, le diclofénac, le naproxène, l'ibuprofène, le célécoxib, l'acide méfénamique, l'étoricoxib ou l'indométacine ; et un agent efficace pour le traitement de la douleur neuropathique, tel qu'un autre agent thérapeutique sélectionné dans le groupe constitué par : les analogues de l'acide gamma-aminobutyrique, tels que la gabapentine ou la prégabaline ; les capsaïcinoïdes, tels que la capsaïcine ; les inhibiteurs de la recapture de la sérotonine et de la noradrénaline, tels que la duloxétine ; les anticonvulsivants, tels que la carbamazépine ou l'oxcarbazépine ; les antidépresseurs tricycliques, tels que l'amitriptyline ou la nortriptyline ; les modulateurs du récepteur du canal cationique à potentiel de récepteur transitoire, sous-famille V, membre 1 (TRPV1), tels qu'un agoniste du récepteur TRPV1 ou un antagoniste du récepteur TRPV1 ; et les modulateurs des récepteurs de la neurokinine, tels que les antagonistes des récepteurs de la neurokinine ou les agonistes des récepteurs de la neurokinine.

13. Composé pour une utilisation selon l'une quelconque des revendications précédentes, dans lequel le composé est capable d'induire chez un sujet une réduction de la douleur d'au moins 5 %, telle qu'au moins 10 %, telle qu'au moins 15 %, telle que 20 % de réduction de la douleur.

14. Composé pour une utilisation selon l'une quelconque des revendications précédentes, dans lequel l'administration du composé est une administration orale.

15. Composé pour une utilisation selon l'une quelconque des revendications précédentes, dans lequel le composé est formulé dans une composition pharmaceutique comprenant en outre un diluant, un support et/ou un excipient pharmaceutiquement acceptable ; par exemple dans lequel le composé est formulé sous une forme galénique solide, telle qu'un comprimé, une gélule, une pilule, des granulés ou une poudre.
